(19) Europäisches Patentamt
European Patent Office
Office européen des brevets

(11) EP 1 798 270 A1

(12) EUROPEAN PATENT APPLICATION
published in accordance with Art. 158(3) EPC

(43) Date of publication:
**20.06.2007 Bulletin 2007/25**

(21) Application number: **05785606.4**

(22) Date of filing: **22.09.2005**

(51) Int Cl.:
*C09K 11/08* (2006.01) *C09K 11/56* (2006.01)
*C09K 11/62* (2006.01) *C09K 11/64* (2006.01)
*C09K 11/70* (2006.01) *C09K 11/74* (2006.01)
*C09K 11/88* (2006.01)

(86) International application number:
**PCT/JP2005/017493**

(87) International publication number:
**WO 2006/033396 (30.03.2006 Gazette 2006/13)**

(84) Designated Contracting States:
**DE FR GB IT**

(30) Priority: **22.09.2004 JP 2004275675**

(71) Applicants:
- **Japan Science and Technology Agency
Kawaguchi-shi,
Saitama 332-0012 (JP)**
- **National University Corporation
Hokkaido University
Sapporo-shi, Hokkaido 0600808 (JP)**

(72) Inventors:
- **JIN, Takashi
Sapporo-shi, Hokkaido 0650020 (JP)**
- **KINJO, Masataka
Sapporo-shi, Hokkaido 0010024 (JP)**
- **TAMURA, Mamoru
Sapporo-shi, Hokkaido 0630001 (JP)**
- **FUJII, Fumihiko
Sapporo-shi, Hokkaido 0600033 (JP)**
- **SAKATA, Hiroshi
Sapporo-shi, Hokkaido 0600002 (JP)**

(74) Representative: **von Kreisler, Alek et al
Patentanwälte
von Kreisler-Selting-Werner
Postfach 10 22 41
D-50462 Köln (DE)**

(54) **WATER-SOLUBLE FLUORESCENT MATERIAL AND METHOD FOR PRODUCING SAME**

(57) The present invention provides a water-soluble fluorescent material having a luminescent region at a visible range and having excellent luminescent efficiency. The present invention relates to a water-soluble fluorescent material having a semiconductor nanocrystal; a linear or cyclic phenol compound having hydrophilic group and hydrophobic group that coats at least a portion of the surface of the semiconductor nanocrystal; and a coordinating organic compound coating at least a portion of the surface of the semiconductor nanocrystal.

Fig. 3

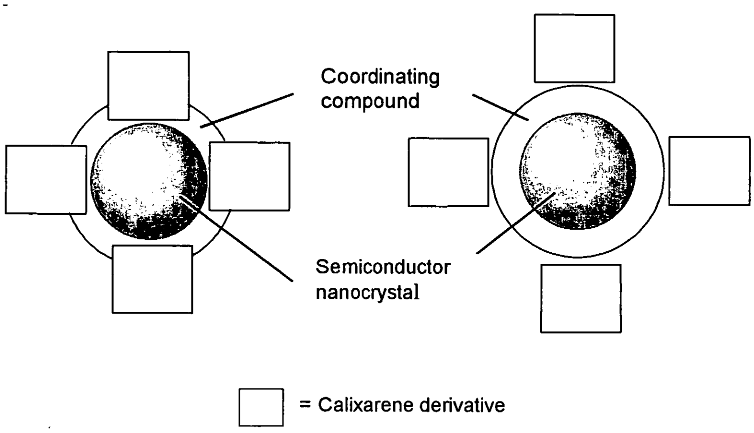




Printed by Jouve, 75001 PARIS (FR)

## Description

### FIELD OF THE INVENTION

The present invention relates to a water-soluble fluorescent material having excellent luminescent efficiency. Specifically, the present invention relates to a water-soluble fluorescent material having a luminescent region at a visible and near-infrared range and having excellent luminescent efficiency.

### BACKGROUND OF THE INVENTION

Luminescent materials are currently used for a display field, a photonic device field, a fluorescent probe for biology and the like. Rare Earth Elements, organic dyes and the like are mentioned as the typical luminescent material.

Rare Earth Elements are mainly used as a glass luminescent material containing the Rare Earth Elements which are dispersed in inorganic glass medium. There is however a problem that luminance of Rare Earth Elements is little, because the luminescent lifetime of the Rare Earth Elements is long in general. Additionally, there is a problem that it is difficult to further enhance the luminance, because absorption saturation occurs easily.

On the other hand, the organic dyes have great advantage that the organic dyes have large luminance because a luminescence lifetime of the organic dyes is short. The wavelength of light absorbed of the organic dyes is however limited to a specific range because the excitation level of the organic dyes is discontinuous.
Thus, it is difficult for the organic dyes to change absorption wavelength in accordance with utilization uses. Additionally, there is also a problem that the molecule of the organic dyes is decomposed by photo irradiation to discolor.

Under these circumstances, semiconductor nanocrystals have been recently marked as a fluorescent material. It is publicly known that the semiconductor nanocrystals exhibit various characteristics which are not observed in the above-mentioned fluorescent material. The semiconductor nanocrystals mean semiconductor particles with a particle diameter of about 1 to 200 nm. Luminescence property of the semiconductor nanocrystals includes large luminance because of having the same luminescence lifetime as that of the organic dyes, as well as extreme stability for discoloration, etc. Furthermore, the semiconductor nanocrystals have characteristic of having a band structure. The semiconductor nanocrystals absorb all of light with shorter wavelengths (high energy) than a band gap of the band structure and emit light with a wavelength depending on the band gap. The band gap depends on the particle diameter of the semiconductor nanocrystals.

The semiconductor nanocrystals are produced by miniaturizing semiconductor by various methods. However, its reactivity of crystal surface is dramatically increased by the miniaturization. Consequently, it is difficult to provide crystals emitting light with a specific wavelength, because the reactivity of crystal surface is heightened.
For these reasons, it has been difficult to use the semiconductor nanocrystal as the luminescent material.

The semiconductor nanocrystals have been marked as the luminescent material and practically used, since the group of Alivisantos and Bawendi in USA succeeded in deactivation by coating the crystal surface of semiconductor with an organic molecule in solution in the latter half of 1990 (X. Peng, M.C. Schlamp and A.P. Alivisatos, J. Am. Chem. Soc. 119, 7019(1997)) and B.O. Dabbousi, J. Rodriguez-Viejo, F.V. Mikulec, J.R. Heine, H. Mattoussi, R. Ober, K.F. Jensen, M.G. Bewendi, J. Phys. Chem. B, 101, 9463 (1997). Then, study for obtaining water-soluble semiconductor crystal has been actively carried out by chemically modifying the surface of the semiconductor nanocrystal with a hydrophilic functional group in order to use the semiconductor nanocrystal as fluorescent probe. The following two types are mentioned as the chemical modification method currently publicly known.

The first type is a method of substituting the hydrophobic coating agent of the crystal surface of semiconductor with a thiol amphiphilic compound to be made water-soluble (W.C.W. Chan, S. Nie, Science, 281, 2016 (1998)) and D. Gerion, F. Pinaud, S.C. Williams, W.J. Parak, D. Zanchet, S. Weiss, A.P. Alivasatos, J. Phys. Chem. B. 105, 8861 (2001)). The schematic illustration drawing of this type is shown in FIG.1.

The first type method is however required to use a thiol reagent having toxicity or corrosiveness in a production step and accordingly, there is a problem that the cost of a production facility and the like is high. Additionally, there is also a problem that the water-soluble semiconductor nanocrystal obtained by the method is low in luminescent efficiency.

The second type method is a method of coating the crystal surface of semiconductor with an amphillic polymer to be made water-soluble (the pamphlet of WO 00/17655, B. Dubertret, P. Skourides, D.J. Norris, V. Noireaux, A.H. Brivanlou, A. Libchaber, Science, 298, 1759(2002)) and S. Kim, Y.T. Lim, E.G. Soltesz, A.M. De Grand, J. Lee, A. Nakayama, J.A. Parfer, T. Mihaljevic, R.G. Laurence, D.M. Dor, L.H. Cohn, M.G. Bawendi, J.V. Frangioni, Nature Biotech. 22, 93(2004)). The schematic illustration drawing of this type is shown in FIG.2.

However, there is a problem that the water-soluble semiconductor nanocrystal obtained by the second type method is also low in luminescent efficiency. Additionally, there are also problems that a polymer coating agent used is precious and the preparation of the water-soluble semiconductor nanocrystal requires long time etc.
Currently, the water-soluble semiconductor nanocrystal (quantum dot) prepared using the polymer coating agent is

commercially available as a fluorescent probe for cell staining. It is very precious in comparison with conventional organic dyes, because preparation is difficult and a reagent used is precious.

The water-solubilization of the two types currently publicly known have any one of problems that the luminescent efficiency of the water-soluble semiconductor crystals obtained is low, safety in synthesis and economical efficiency (the difficulty in preparation of the reagent used, high price, long time is consumed for preparation and the like), or the convenience of preparation.

Experimental error by size and the deactivation of those labeled become occasionally a problem depending on the measurement objective, because a size of the semiconductor nanocrystal is large in comparison with the conventional organic dyes. Furthermore, the semiconductor nanocrystal has characteristic that the wavelength of light emitted is determined depending on their sizes. It is necessary for preparing the semiconductor nanocrystal having luminescent wavelength at long wavelength side that crystals with larger particle diameters are prepared. In this case, the above-mentioned problems are further serious and the application uses of the semiconductor nanocrystal are limited.

## SUMMARY OF THE INVENTION

It is an object of the present invention to solve the above-mentioned problems of conventional technologies. More specifically, it is an object of the present invention to provide a water-soluble fluorescent material having excellent luminescent efficiency and to provide a method of simply preparing it.

The present invention provides a water-soluble fluorescent material having a semiconductor nanocrystal, a linear or cyclic phenol compound having hydrophilic group and hydrophobic group that coats at least a portion of the surface of the semiconductor nanocrystal and a coordinating organic compound coating at least a portion of the surface of the semiconductor nanocrystal, and the above-mentioned objects are attained thereby.

The semiconductor nanocrystal has preferably a semiconductor nanocrystal core and a shell layer coating the semiconductor nanocrystal core.

Furthermore, the above-mentioned coordinating organic compound is one or more compound selected from a group consisting of trialkylphosphines, trialkylphosphineoxides and ω-aminoalkanes.

Furthermore, an aspect of the linear or cyclic phenol compound having hydrophilic group and hydrophobic group includes one or more calixarene derivatives indicated by the following formula (I):

[Chem. 1]

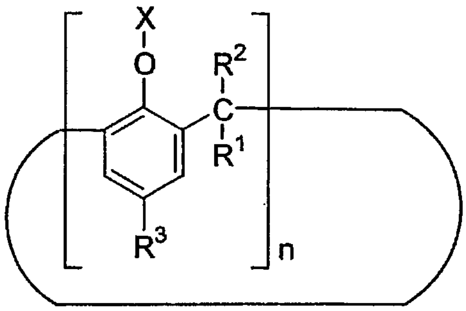

(I)

wherein,

X represents H, $CH_2)_mCOOH$, $(CH_2)_mOH$, $(CH_2)_mNH_2$, $(CH_2)_mN(CH_3)_3$, $(CH_2)_mPO_4H_2$, $(CH_2)_mSO_3H$, $(CH_2)_mCHO$, $\{(CH_2)_2O\}_m(CH_2)_2OH$ or a salt thereof, wherein m is an integer of 1 to 5 and X may be bonded with other phenolic oxygen atom to form a crown ether with a 3- to 30-membered ring,

each of $R^1$ and $R^2$ independently represents H, $CH_3$, $C_2H_5$ or $C_3H_7$, $R^3$ represents H or linear or branched $C_1$ to $C_{12}$-alkyl, $C_3$ to $C_{12}$-cycloalkyl, $C_6$ to $C_{12}$-aryl or $C_7$ to $C_{12}$-aralkyl,

n is an integer of 4 to 20 and X may be the same or different,

or indicated by the following formula (II):

[Chem. 2]

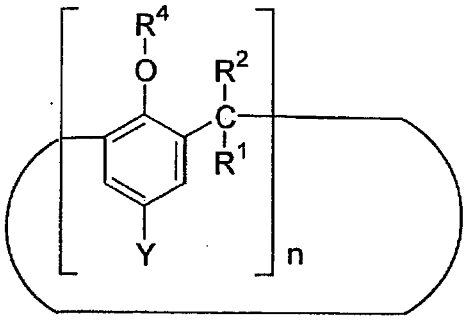

(II)

wherein,
Y represents $(CH_2)_tCOOH$, $(CH_2)_tOH$, $(CH_2)_tNH_2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5, each of $R^1$ and $R^2$ independently represents H, $CH_3$, $C_2H_5$ or $C_3H_7$, $R^4$ represents linear or branched $C_1$ to $C_{12}$-alkyl, $C_3$ to $C_{12}$-cycloalkyl, $C_6$ to $C_{12}$-aryl or $C_7$ to $C_{12}$-aralkyl, n is an integer of 4 to 20 and Y may be the same or different.

Furthermore, another aspect of the linear or cyclic phenol compound having hydrophilic group and hydrophobic group includes one or more thiacalixarene derivatives indicated by the following formula (III):

[Chem. 3]

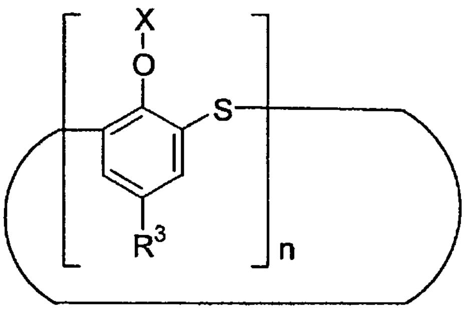

(III)

wherein,
X represents H, $(CH_2)_mCOOH$, $(CH_2)_mOH$, $(CH_2)_mNH_2$, $(CH_2)_mN(CH_3)3$, $(CH_2)_mPO_4H_2$, $(CH_2)_mSO_3H$, $(CH_2)_mCHO$, $\{(CH_2)_2O\}_m(CH_2)_2OH$ or a salt thereof, wherein m is an integer of 1 to 5 and X may be bonded with other phenolic oxygen atom to form a crown ether with a 3- to 30-membered ring,
$R^3$ represents H or linear or branched $C_1$ to $C_{12}$-alkyl, $C_3$ to $C_{12}$-cycloalkyl, $C_6$ to $C_{12}$-aryl or $C_7$ to $C_{12}$-aralkyl, n is an integer of 4 to 20 and X may be the same or different,
or indicated by the following formula (IV):

[Chem. 4]

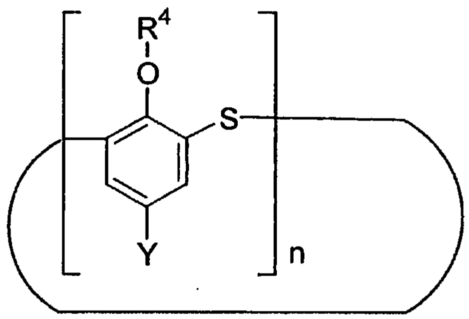

(IV)

wherein,
Y represents $(CH_2)_tCOOH$, $(CH_2)_tOH$, $(CH_2)_tNH_2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5,
$R^4$ represents linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and Y may be the same or different.

Furthermore, another aspect of the linear or cyclic phenol compound having hydrophilic group and hydrophobic group includes one or more calixresorcarene derivatives indicated by the following formulae:

[Chem. 5]

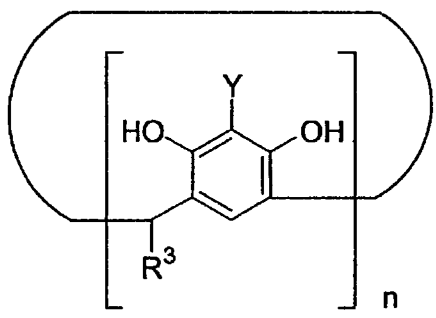

(V)

[Chem. 6]

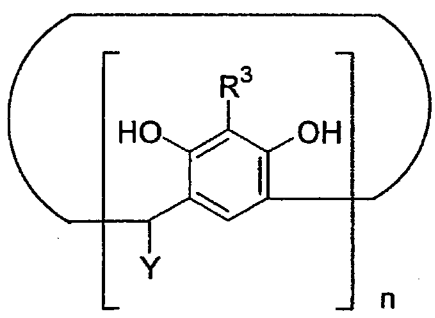

(VI)

[Chem. 7]

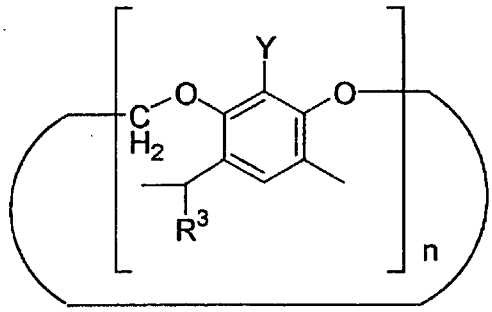

(VII)

[Chem. 8]

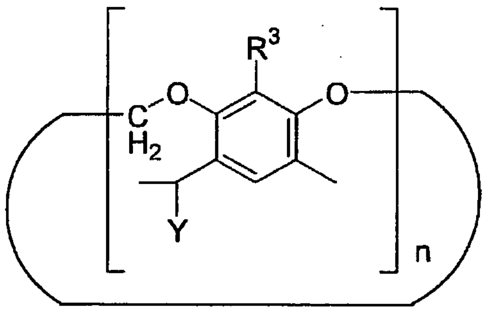

(VIII)

in the formulae (V) to (VIII),
Y represents $(CH_2)_tCOOH$, $(CH_2)_tOH$, $(CH_2)_tNH_2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5,
$R^3$ represents H or linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and Y may be the same or different.

Furthermore, another aspect of the linear or cyclic phenol compound having hydrophilic group and hydrophobic group includes one or more linear phenol compound derivatives indicated by the following formula (IX):

[Chem. 9]

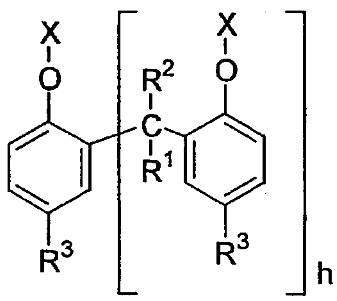

(IX)

wherein,
X represents H, $(CH_2)_mCOOH$, $(CH_2)_mOH$, $(CH_2)_mNH_2$, $(CH_2)_mN(CH_3)_3$, $(CH_2)_mPO_4H_2$, $(CH_2)_mSO_3H$, $(CH_2)_mCHO$,

$\{(CH_2)_2O\}_m(CH_2)_2OH$ or a salt thereof, wherein m is an integer of 1 to 5 and X may be bonded with other phenolic oxygen atom to form a crown ether with a 3- to 30-membered ring,
each of $R^1$ and $R^2$ independently represents H, $CH_3$, $C_2H_5$ or $C_3H_7$, $R^3$ represents H or linear or branched $C_1$ to $C_{12}$-alkyl, $C_3$ to $C_{12}$-cycloalkyl, $C_6$ to $C_{12}$-aryl or $C_7$ to $C_{12}$-aralkyl,
h is an integer of 0 to 99 and if h is 1 or more, X may be the same or different,
or indicated by the following formula (X):

[Chem. 10]

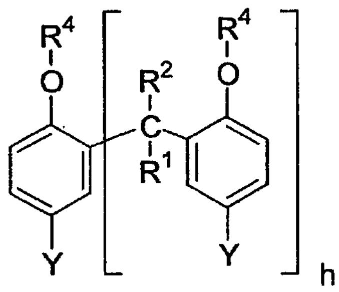

(X)

wherein,
Y represents $(CH_2)_tCOOH$, $(CH_2)_tOH$, $(CH_2)_tNH_2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5,
each of $R^1$ and $R^2$ independently represents H, $CH_3$, $C_2H_5$ or $C_3H_7$, $R^4$ represents linear or branched $C_1$ to $C_{12}$-alkyl, $C_3$ to $C_{12}$-cycloalkyl, $C_6$ to $C_{12}$-aryl or $C_7$ to $C_{12}$-aralkyl,
h is an integer of 0 to 99 and if h is 1 or more, Y may be the same or different.

The semiconductor nanocrystal core comprises preferably Group II-VI compound semiconductor, Group III- V compound semiconductor or Group IV semiconductor.

Furthermore, the shell layer preferably includes one or more semiconductors selected from a group consisting of ZnO, ZnS, ZnSe, ZnTe, CdO, CdS, CdSe, CdTe, MgS, MgSe, GaAs, GaN, GaP, GaAs, GaSb, HgO, HgS, HgSe, HgTe, InAs, InN, InP, InSb, AlAs, AlN, AlP and AlSb, or an alloy or mixed crystal thereof.

Furthermore, the semiconductor nanocrystal core preferably includes CdSe or CdTe, or a mixture, an alloy or mixed crystal thereof, and the shell layer preferably includes ZnS.

The present invention also provides a method for preparing a water-soluble fluorescent material. The method includes a step of mixing the semiconductor nanocrystal whose surface is coated with a coordinating organic compound, with a linear or cyclic phenol compound having hydrophilic group and hydrophobic group in solvent.

In the mixing step, the semiconductor nanocrystal preferably has a semiconductor nanocrystal core and a shell layer coating the semiconductor nanocrystal core.

Furthermore, in the mixing step, the linear or cyclic phenol compound having hydrophilic group and hydrophobic group used in the mixing step is preferably one or more calixarene derivatives indicated by the above-mentioned formula (I) or (II) ;
or one or more thiacalixarene derivatives indicated by the above-mentioned formula (III) or (IV);
or
one or more calixresorcarene derivatives indicated by the above-mentioned formulae (V), (VI), (VII) or (VIII);
or
one or more linear phenol compound derivatives indicated by the above-mentioned formula (IX) or (X).

In the mixing step, the semiconductor nanocrystal core preferably includes Group II-VI compound semiconductor, Group III- V compound semiconductor or Group IV semiconductor.

Furthermore, in the mixing step, the shell layer preferably includes one or more semiconductors selected from a group consisting of ZnO, ZnS, ZnSe, ZnTe, CdO, CdS, CdSe, CdTe, MgS, MgSe, GaAs, GaN, GaP, GaAs, GaSb, HgO, HgS, HgSe, HgTe, InAs, InN, InP, InSb, AlAs, AlN, AlP and AlSb, or an alloy or mixed crystal thereof.

Furthermore, in the mixing step, the semiconductor nanocrystal core comprise preferably CdSe or CdTe or a mixture, an alloy or mixed crystal thereof, and the shell layer includes ZnS.

Furthermore, an ultrasonic process is preferably used in the mixing step.

Furthermore, the method can be carried out for obtaining the water-soluble fluorescent material having a

luminescent region at visible and near-infrared range. Furthermore, the method can be carried out for obtaining the water-soluble fluorescent material having a luminescent region at visible range.

The present invention also provides the water-soluble fluorescent material obtainable from the above-mentioned process.

Furthermore, the present invention also provides use of the linear or cyclic phenol compound having hydrophilic group and hydrophobic group in the preparation of the above-mentioned water-soluble fluorescent material.

Furthermore, the present invention provides a method for preparing the water-soluble fluorescent material changing a luminescent wavelength, including a step of mixing a semiconductor nanocrystal whose surface is coated with a coordinating organic compound, with a linear or cyclic phenol compound having hydrophilic group and hydrophobic group in solvent.

The water-soluble fluorescent material can be prepared safely, simply and speedily by the present invention without requiring the use of a coating agent with toxicity and corrosiveness represented by thiol compounds conventionally used. The water-soluble fluorescent material of the present invention has significantly high luminescent efficiency in comparison with conventional water-soluble semiconductor nanocrystal. For example, the water-soluble fluorescent material according to the present invention has high luminescent efficiency by 10-fold in comparison with the conventional water-soluble semiconductor nanocrystal using mercaptopropionic acid. Furthermore, the water-soluble fluorescent material of the present invention has also advantage superior in dispersion stability. The present invention can be basic water-solubilization technology for applying the semiconductor nanocrystal as a fluorescent probe and a luminescent material.

## BRIEF DESCRIPTION OF THE DRAWINGS

FIG.1 is a schematic illustration diagram of a conventional technology;
FIG.2 is a schematic illustration diagram of a conventional technology;
FIG.3 is a schematic diagram of the water-soluble fluorescent material of the present invention;
FIG.4 is a schematic illustration diagram of the basic structure of a device used for detection and measurement by FCS;
FIG.5 is a graph showing a shift of luminescence wavelength by the water-soluble fluorescent material of the present invention;
FIG.6 is a graph showing a fluorescent intensity of the water-soluble fluorescent material of Example 1, the water-soluble nanocrystal of Comparative Example 1 and CdSe (ZnS) coated with TOPO;
FIG.7 is a graph showing a fluorescent intensity of the water-soluble fluorescent material of Example 1, the water-soluble nanocrystal of Comparative Example 1 and CdSe (ZnS) coated with TOPO;
FIG.8 is a graph related to a particle diameter measurement of the water-soluble fluorescent material of the present invention by detection and measurement by FCS; and
FIG.9 is a graph showing a change of dispersion time before and after labeling GFP antibody.

## DETAILED DESCRIPTION OF THE PREFERRED EMBODIMENTS

The water-soluble fluorescent material of the present invention has a structure in which at least a portion of a surface of the semiconductor nanocrystal is coated with a linear or cyclic phenol compound having hydrophilic group and hydrophobic group, and other portion of the surface of the semiconductor nanocrystal is coated with a coordinating organic compound.

### Semiconductor nanocrystal

The semiconductor nanocrystal contained in the water-soluble fluorescent material of the present invention is a semiconductor nanocrystal having a luminescent band at a visible range or its surrounding. As the semiconductor nanocrystal, a semiconductor having a semiconductor nanocrystal core and a shell layer coating the semiconductor nanocrystal core (so-called core shell structure) is preferably used.

Group II-VI compound semiconductor, Group III- V compound semiconductor or Group IV semiconductor is preferably used as the semiconductor nanocrystal core, because these semiconductors have luminescent bands at a visible region or it surrounding. Group III- V compound semiconductor includes, for example, GaN, GaP, GaAs, GaSb, InN, InP, InAs, InSb, AlAs, AlP, AlSb, AlS and the like. Group II-VI compound semiconductor includes, for example, ZnO, ZnS, ZnSe, ZnTe, CdO, CdS, CdSe, CdTe, HgO, HgS, HgSe, HgTe and the like. Group IV semiconductor includes, for example, Ge, Si, Pb and the like. These semiconductors may be used alone and 2 or more may be used. Furthermore,

these semiconductors may be an alloy comprising 2 or more of semiconductors or a mixed crystal.

The Group II-VI compound semiconductor is more preferably used as the semiconductor nanocrystal core. CdSe or CdTe or a mixture thereof, alloy or mixed crystal is most preferably used, because these semiconductors are superior in the controllability of particle diameter of crystal and have high luminescent performance.

As the preparation method of the semiconductor nanocrystal core, for example, the following methods are mentioned.

(1) A method of letting raw material aqueous solution exist as inverted micelle in nonpolar organic solvent to grow crystal structure in the inverted micelle phase (inverted micelle method).
For example, the mehod is described in B.S. Zou et al, Int. J. Quant. Chem. Vol.72, 439 (1999), and is a publicly known method. It is suitable for industrial production, because comparatively cheap and chemically stable salt can be used as a raw material and furthermore, the method is carried out at comparatively low temperature that does not exceed the boiling point of water. However, the method is occasionally inferior in luminescent property at current technology in comparison with the case of the following hot soap method.

(2) A method of injecting a thermally degradable raw material in liquid phase organic medium at high temperature to grow crystal structure (hot soap method).
For example, the method is a publicly known method described in a literature edited by J.E.B. Katari et. al. The method has characteristics that semiconductor crystal particles superior in particle diameter distribution and purity in comparison with the above-mentioned inverted micelle method are obtained and the obtained product is superior in luminescent property and usually soluble in organic solvent. A coordinate organic compound with suitable coordinating force for semiconductor-constituting element is selected as a liquid phase component (doubling with a solvent and a ligand) in order to desirably control reaction speed of crystal growth in liquid phase in the hot soap method. The example of the coordinate organic compound includes trialkylphosphines, trialkylphosphine oxides and ω-aminoalkanes such as dodecylamine, tetradecylamine, hexadecylamine and octadecylamine. Among these, trialkylphosphine oxides such as trioctylphosphine oxide (TOPO), ω-aminoalkanes such as hexedecylamine, and the like are preferable.

(3) A method suitable for industrial production by which semiconductor crystal and its precursor are prepared in protonic solvents such as water and ethanol at comparatively low temperature of 100°C or less, using acid-base reaction as driving force (sol-gel method).

Among the above-mentioned preparation methods, the hot soap method (2) is preferably used. The reason is that the method can provide the semiconductor nanocrystal core more superior in luminescent property. Furthermore, a method for preparing the semiconductor nanocrystal coated with trialkylphosphine oxides is also described in L. Qum and X. Peng, J. Am. Chem. Soc., Vol.124, 2049(2002).

The semiconductor nanocrystal core preferably has a particle diameter of about 1 nm to about 50 nm, more preferably about 1 nm to about 20 nm, and further preferably about 1 nm to about 5 nm. The water-soluble fluorescent material having good luminescent property can be obtained by having a particle diameter within the above-mentioned range.

The shell layer coating the semiconductor nanocrystal core contains a semiconductor whose band gap (forbidden band width) is larger than the band gap of the semiconductor nanocrystal core. Energetic barrier is formed in semiconductor nanocrystal by using the semiconductor as the shell layer and good luminescent performance is obtained thereby.

The semiconductor preferably used for the shell layer may depend on the band gap of the semiconductor nanocrystal core used, for example, one or more semiconductors selected from a group consisting of ZnO, ZnS, ZnSe, ZnTe, CdO, CdS, CdSe, CdTe, MgS, MgSe, GaAs, GaN, GaP, GaAs, GaSb, HgO, HgS, HgSe, HgTe, InAs, InN, InP, InSb, AlAs, AlN, AlP and AlSb, or an alloy or a mixed crystal thereof is preferably used. Among these, ZnS is preferably used. The semiconductor nanocrystal having a core-shell structure can be easily prepared by using ZnS.

The semiconductor nanocrystal having a core-shell structure is preferably prepared by a two-stage method. The shell layer coating the semiconductor nanocrystal core can be formed by adding a solution of semiconductor contained in the shell layer, to the semiconductor nanocrystal core that was obtained by the above-mentioned method. The preparation method is described in B. O. Dabbousi, J. Rodriguez-Viejo, F.V. Mikulec, J. R. Heine, H. Mattoussi, R. Ober, K.F. Jensen, M. G. Bawendi, J. Phys. Chem. B, 101, 9463(1997) and the like and is publicly known.

The semiconductor nanocrystal having a core-shell structure preferably has a particle diameter of about 1 nm to about 100 nm, more preferably about 1 nm to about 40 nm and further preferably about 1 nm to about 10 nm. A luminescent area at a visible range can be obtained by having a particle diameter within the above-mentioned range.

Commercially available semiconductor nanocrystal may be used. The semiconductor nanocrystal preferably used includes Core-Shell Evidots (trade name) (CdSe/ZnS and CdTe/CdS semiconductor crystal) manufactured by Evident Technologies Inc. and the like.

In the present invention, the semiconductor nanocrystal whose surface is coated with a coordinating organic compound is used. The surface of the semiconductor nanocrystal is generally coated with the coordinating organic compound such as trioctylphosphine oxide (TOPO), because the reactivity of the surface of the semiconductor nanocrystal is very high. The semiconductor nanocrystal thus coated has very strong hydrophobic property and can be dispersed only in organic solvents such as chloroform and hexane. The present invention makes it possible to disperse in water the semiconductor nanocrystal coated with the coordinating organic compound which cannot be dispersed in water. The example of the coordinating organic compound includes trialkylphosphine oxides such as trioctylphosphine oxide (TOPO), trialkylphosphines such as trioctylphosphine (TOP) and tributylphosphine (TBP) or ω-aminoalkanes such as hexadecylamine, and the like.

Furthermore, in the present invention, the core-shell type semiconductor nanocrystal is preferably used because of its excellent luminescent performance, but it is no limited for the core-shell type semiconductor nanocrystal so far as the semiconductor nanocrystal has excellent luminescent performance. For example, a dope type semiconductor nanocrystal and the like may be used.

Linear or cyclic phenol compound having hydrophilic group and hydrophobic group

In the water-soluble fluorescent material of the present invention, at least one portion of the surface of the semiconductor nanocrystal is coated with the linear or cyclic phenol compound having hydrophilic group and hydrophobic group (hereinafter, occasionally abbreviated as the "phenol compound").

The example of such a phenol compound includes, for example, calixarene derivatives, thiacalixarene derivatives, calixresorcarene derivatives and linear phenol compound derivatives. The calixarene derivatives, thiacalixarene derivatives and calixresorcarene derivatives are compounds having a columnar structure and are known that they have enclosure function and catalyst function.

As the calixarene derivatives, those indicated by the following formula (I) or (II) are mentioned.

[Chem. 11]

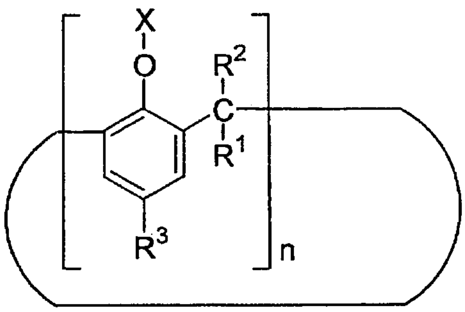

(I)

wherein,
X represents H, $(CH_2)_mCOOH$, $(CH_2)_mOH$, $(CH_2)_mNH_2$, $(CH_2)_mN(CH_3)_3$, $(CH_2)_mPO_4H_2$, $(CH_2)_mSO_3H$, $(CH_2)_mCHO$, $\{(CH_2)_2O\}_m(CH_2)_2OH$ or a salt thereof, wherein m is an integer of 1 to 5 and X may be bonded with other phenolic oxygen atom to form a crown ether with a 3- to 30-membered ring,
each of $R^1$ and $R^2$ independently represents H, $CH_3$, $C_2H_5$ or $C_3H_7$, $R^3$ represents H or linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and X may be the same or different.
For example, in a case that X is the form of a salt, X represents $(CH_2)_mCOOH$ or $(CH_2)_mSO_3H$, and when these are salts, $Li^+$, $Na^+$, $K^+$ and the like are mentioned as the counter ion. Furthermore, when X represents $(CH_2)_mNH_2$ and these are salts, for example, $F^-$, $Cl^-$, $Br^-$, $OH^-$, $CH_3COO^-$ and the like are mentioned as the counter ion. Furthermore, when X represents $(CH_2)_mPO_4H_2$ and these are salts, for example, $Li^+$, $Na^+$, $K^+$, $Mg^{2+}$, $Ca^{2+}$ and the like are mentioned as the counter ion.

[Chem. 12]

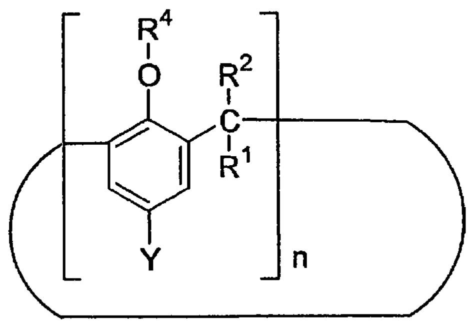

(II)

wherein, Y represents $(CH_2)_tCOOH$, $(CH_2)_tOH$, $(CH_2)_tNH_2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5,
each of $R^1$ and $R^2$ independently represents H, $CH_3$, $C_2H_5$ or $C_3H_7$, $R^4$ represents linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and Y may be the same or different.
For Example, in a case that Y is the form of a salt, Y represents $(CH_2)_tCOOH$ or $(CH_2)_tSO_3H$, and when these are salts, for example, $Li^+$, $Na^+$, $K^+$ and the like are mentioned as the counter ion. Furthermore, when Y represents $(CH_2)_tNH_2$ and these are salts, for example, $F^-$, $Cl^-$, $Br^-$, $OH^-$, $CH_3COO^-$ and the like are mentioned as the counter ion. Furthermore, when Y represents $(CH_2)_tPO_4H_2$ and these are salts, for example, $Li^+$, $Na^+$, $K^+$, $Mg^{2+}$, $Ca^{2+}$ and the like are mentioned as the counter ion.

The $C_1$ to $C_{12}$-alkyl, $C_3$ to $C_{12}$-cycloalkyl, $C_6$ to $C_{12}$-aryl or $C_7$ to $C_{12}$-aralkyl of $R^3$ in the above-mentioned formula (I) and $R^4$ in the above-mentioned formula (II) may have 1 to 4 of substituents such as fluorine, chlorine or bromine.

Among the calixarene derivatives indicated by the above-mentioned formula (I), those in which X represents H, $(CH_2)_mCOOH$, $(CH_2)_mSO_3H$ or a salt thereof, wherein m is an integer of 1 to 3, each of $R^1$ and $R^2$ represents H, $R^3$ represents H or linear or branched $C_1$ to $C_{12}$-alkyl, $C_3$ to $C_{12}$-cycloalkyl, $C_6$ to $C_{12}$-aryl or $C_7$ to $C_{12}$-aralkyl, n is an integer of 4 to 8 and X is the same are more preferable.
In case that X is the form of a salt, $Na^+$ or $K^+$ is preferred as the counter ion.

Furthermore, among the calixarene derivatives indicated by the above-mentioned formula (II), those in which Y represents $(CH_2)_tCOOH$, $(CH_2)_tSO_3H$ or a salt thereof,
wherein t is an integer of 0 to 2, each of $R^1$ and $R^2$ represents H, $R^4$ represents linear or branched $C_1$ to $C_{12}$-alkyl, $C_3$ to $C_{12}$-cycloalkyl, $C_6$ to $C_{12}$-aryl or $C_7$ to $C_{12}$-aralkyl, n is an integer of 4 to 8 and Y is the same are more preferable. In case that Y is the mode of a salt, $Na^+$ or $K^+$ is preferred as the counter ion.

As the thiacalixarene derivatives, those indicated by the following formula (III) or (IV) are mentioned.

[Chem. 13]

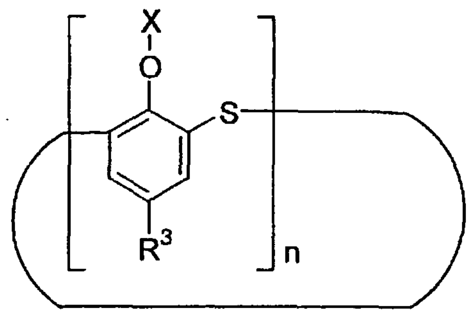

(III)

[Chem. 14]

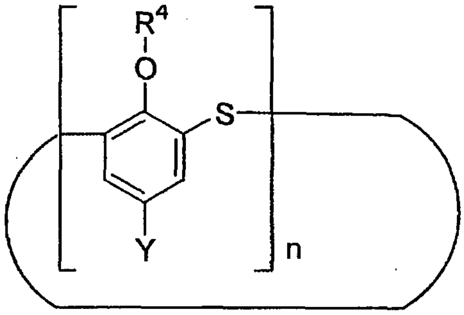

(IV)

The definitions and preferable ranges of $R^3$, X and n in the above-mentioned formula (III) are similar as the definitions and preferable ranges in the above-mentioned formula (I). Furthermore, the definitions and preferable ranges of $R^4$, Y and n in the above-mentioned formula (IV) are similar as the definitions and preferable ranges in the above-mentioned formula (II).

As the calixresorcarene derivatives, those indicated by the following formulae (V) to (VIII) are mentioned.

[Chem. 15]

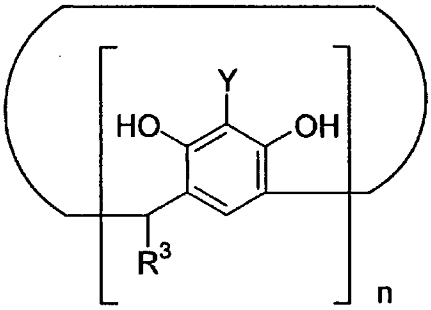

(V)

[Chem. 16]

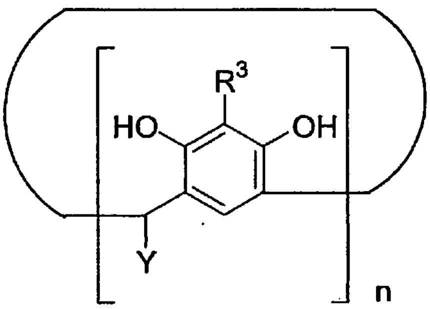

(VI)

[Chem. 17]

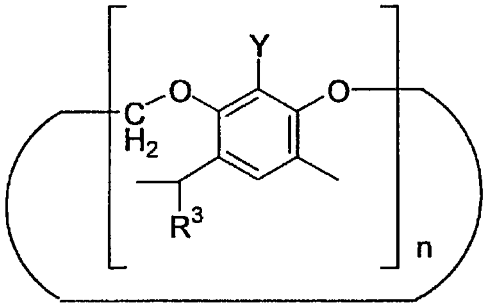

(VII)

[Chem. 18]

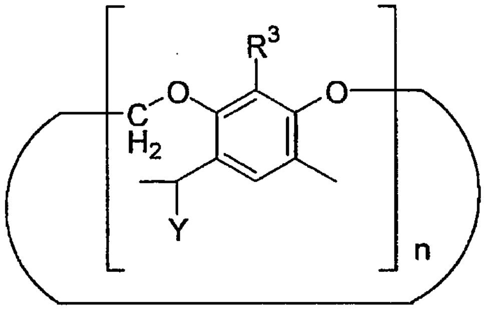

(VIII)

The definitions and preferable ranges of $R^3$ and n in the above-mentioned formulae (V) to (VIII) are similar as the definitions and preferable ranges in the above-mentioned formula (I). Furthermore, the definition and preferable range of Y in the above-mentioned formulae (V) to (VIII) are similar as the definition and preferable range in the above-mentioned formula (II).

As the linear phenol compound derivatives, those indicated by the following formula (IX) or (X) are mentioned.

[Chem. 19]

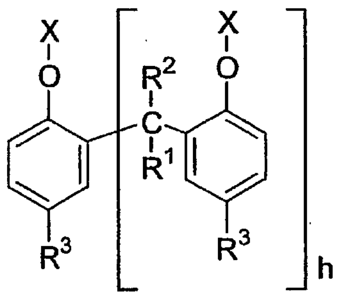

(IX)

[Chem. 20]

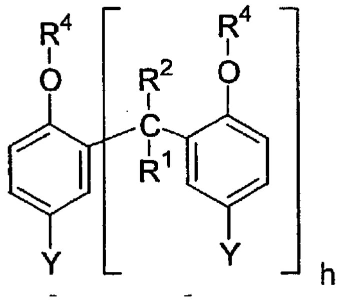

(X)

The definitions and preferable ranges of $R^1$, $R^2$, $R^3$, X and n in the above-mentioned formula (IX) are similar as the definitions and preferable ranges in the above-mentioned formula (I). Furthermore, the definitions and preferable ranges of $R^1$, $R^2$, $R^4$, Y and n in the above-mentioned formula (X) are similar as the definitions and preferable ranges in the above-mentioned formula (II). h in the above-mentioned formulae (IX) and (X) is an integer of 0 to 99, preferably an integer of 0 to 50 and more preferably an integer of 0 to 10.

In the present invention, the calixarene derivatives are preferably used as the phenol compound. Furthermore, as the calixarene derivatives preferably used in particular in the present invention, those indicated in the following formulae (I-1) and (II-1) are mentioned. The calixarene derivatives having the following structure have excellent water-solubilization ability and their precursor compounds are easily available.

[Chem. 21]

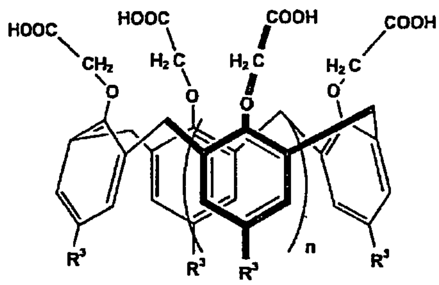

n = 1, 2, 3, 4, or 5

$R^3$ = H or $C(CH_3)_3$

(I-1)

[Chem. 22]

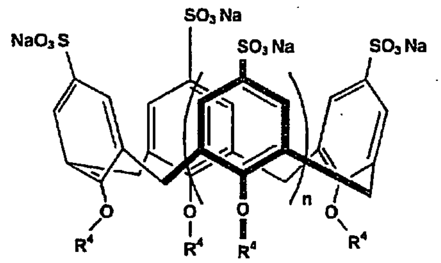

n = 1, 2, 3, 4, or 5

$R^4 = (CH_2)_5CH_3$

(TT-1)

Preparation methods of the calixarene derivatives are described in, for example, J. Am. Chem. Soc., (1989), 111, 8192 (the synthesis method of p-tert-butylcalix-(5)-arene is described), Macromol. Chem. Rapid Commun. 3, 705, (1982) (the synthesis method of p-tert-butylcalix-(7)-arene is described), J. Org. Chem. (1977), 42, 382 and J. Org. Chem. (1978), 43, 4280 (the synthesis method of p-tert-butylcalix-(4)-arene is described) and the like and is publicly known. The calixarene derivatives used in the present invention can be prepared based on these methods. Furthermore, the thiacalixarene derivatives, calixresorcarene derivatives and linear phenol compound derivatives can be prepared by changing a starting substance and using the similar methods.

In the present invention, commercially available phenol compounds may be used. The example of the calixarene derivatives (precursor compound) preferably used include 4-tert-butylcalix[4]arene tetraacetic acid tetraethyl ester manufactured by Sigma-Aldrich Co., 4-sulfonic calix[4]arene manufactured by Acros Organics Co. and the like. The example of the thiacalixarene derivatives (precursor compound) preferably used include 4-tert-butyl-1-(ethoxycarbonylmethoxy) tetra thiacalix[4]arene, 4-sulfothiacalix[4]arene manufactured by Tokyo Chemical Inductry Co., Ltd. and the like. The example of the calixresorcarene derivatives (precursor compound) preferably used include C-undecylcalix[4]resorcinarene monohydrate manufactured by Acros Organics Co. and the like. The example of the linear phenol compound derivatives (precursor compound) preferably used include phenoxyacetic acid, p-phenolsulfonic acid manufactured by Wako Pure Chemical Industries Ltd., and the like. Either of the above-mentioned commercially available compounds may be used as a precursor compound. The phenol compound used in the present invention can be easily prepared by treating the precursor compounds using alkali (for example, sodium hydroxide and the like), or by reacting with alkyl bromide. Furthermore, the alkali treatment is a publicly known treatment method described in A. Aruduini, A. Pochini, S. Reverberi, R. Ungaro, J. Chem. Soc. Commun. 981-982 (1984). The reaction with alkyl bromide is also a publicly known method described in S.Shinkai, S. Mori, H. Koreishi, T. Tsubaki, O. Manabe, J. Am. Chem. Soc. 108, 2409-2416 (1986).

Furthermore, these phenol compounds may be used alone and 2 or more may be used in mixture. Furthermore, if a hydrophilic group such as carboxyl group or hydroxyl group in these phenol compounds is required to be ionized, they may be ionized using a deprotonating agent such as potassium t-butoxide, dimethylaminopyridine and the like.

Water-soluble fluorescent material

The water-soluble fluorescent material of the present invention has a structure in which at least one portion of the surface of the above-mentioned semiconductor nanocrystal is coated with the phenol compound and at least other portion of the surface of the semiconductor nanocrystal is coated with a coordinating organic compound. Namely, the water-soluble fluorescent material of the present invention has a portion of the surface of the semiconductor nanocrystal coated with the phenol compound and a portion coated with a coordinating organic compound. As the structure of the water-soluble fluorescent material of the present invention, one or more the phenol compounds probably coordinate or adhere for the semiconductor nanocrystal coated with the coordinating organic compound. Although it is not limited and not constrained by theory, when the phenol compound is directed or adheres to the semiconductor nanocrystal, the phenol compound is probably substituted with the coordinating organic compound coating the semiconductor nanocrystal, or is inserted into the coordinating organic compound coating the semiconductor nanocrystal, or is directed and adheres on a hydrophobic monomolecular layer comprising the coordinating organic compound coating the semiconductor nanocrystal. Thus, the water-soluble fluorescent material having a structure in which at least one portion of the surface of the semiconductor nanocrystal is coated with the phenol compound and at least other portion of the surface of the semiconductor nanocrystal is coated with the coordinating organic compound is obtained.

When the phenol compound is directed or adheres to the semiconductor nanocrystal, the hydrophobic group possessed by the phenol compound is probably directed or adheres to the semiconductor nanocrystal. For example, when the calixarene derivatives indicated by the above-mentioned formula (I) coats the semiconductor nanocrystal, the group side indicated by $R^3$ being a hydrophobic group is directed or adheres to the semiconductor nanocrystal and the group side indicated by X being a hydrophilic group is faced to outside (solvent side). Furthermore, when the calixarene derivatives indicated by the above-mentioned formula (II) coats the semiconductor nanocrystal, the group side indicated by $R^4$ being a hydrophobic group is directed or adheres to the semiconductor nanocrystal and the group side indicated by Y being a hydrophilic group is faced to outside (solvent side). Since the hydrophilic group is faced to solvent side, the water-soluble fluorescent material is obtained. The orientation and adherence of cases that the thiacalixarene derivatives indicated by the formulae (III) and (IV), calixresorcarene derivatives indicated by the formulae (V) to (VIII) and linear phenol compound derivatives indicated by the formulae (IX) and (X) are used are also similar.

FIG.3 shows the schematic diagram of the water-soluble fluorescent material of the present invention. This is an example of the water-soluble fluorescent material of the present invention. The water-soluble fluorescent material whose a calixarene derivative coats at least a portion of the surface of the semiconductor nanocrystal is schematically shown.

The water-soluble fluorescent material of the present invention has preferably a particle diameter of about 1 nm to about 100 nm, more preferably about 1 nm to about 50 nm and further preferably about 1 nm to about 20 nm. The particle diameter within the above-mentioned range can provide excellent luminescent property.

The measurement of the diameter of the water-soluble fluorescent material according to the present invention is determined using fluorescent correlation spectroscopy (FCS). The fluorescent correlation spectroscopy is a method of evaluating the size of molecule from the autocorrelation of fluctuation of fluorescent intensity. There is advantage that the size of the water-soluble fluorescent material including also a coating agent can be evaluated by measuring the particle diameter of the water-soluble fluorescent material using the fluorescent correlation spectroscopy (FCS). For example, the size of the semiconductor nanocrystal composing the water-soluble fluorescent material can be measured by transmission electron microscope (TEM) that observes an image directly, but it is nearly impossible to measure the size of the whole water-soluble fluorescent material. The fluorescent correlation spectroscopy (FCS) is simply illustrated.

FIG.4 shows the basic structure of a device used for detection and measurement by FCS. Schematically illustrating it using FIG. 4, (A) in FIG.4 shows the schematic diagram of the FCS (fluorescent correlation spectroscopy) device. Excited light from laser is conducted to sample solution placed on a cover glass through a dichroic mirror (DM) and an objective lens. Fluorescent luminescence passes a long pass filter (F), removes background light other than confocal plane by pinhole on the confocal and is conducted to avalanche photodiode detector (APD), and its signal is further analyzed with a digital correlator. (B) in FIG.4 shows the magnified schematic diagram of observation area. There is shown an aspect in which fluorescent molecules under Brown motion pass the confocal area focused by the objective lens to limitation. (C) in FIG.4 shows a correlation curve after fluorescent correlation analysis. Physical quantities such as the "number" and "sizes" of molecules can be obtained by analyzing the fluctuation of fluorescent intensity observed.

Fluorescence from the extremely minute area (a diameter of about 400 nm, an axis length of about 2 $\mu$m and a volume of about $10^{-16}$ L) of sample solution is detected in measurement by FCS by using confocal optics (FIG.4). As the FCS measurement device, for example, ConfoCor 2 manufactured by Carl Zeiss, MF20 manufactured by manufactured Olympus Corporation or Compact FCS by Hamamatsu Photonics K.K. can be used.

Since the observing area is an open system in measurement by FCS, fluorescent molecules move in and out the observing area in accordance with the Brown motion. Then, the number of molecules in the observing area is fluctuated centering around a certain value and the fluctuation of the number is generated. Then, the fluctuation of fluorescent intensity originated in the fluctuation of the number is observed (Protein, Nucleic Acid And Enzyme, Vol.9, 1431-1438 (1999)).

Physical quantities such as the "number" and "sizes" of molecules can be determined by analyzing the fluctuation of fluorescent intensity observed, using the mathematical formulae (1) to (4). Namely, autocorrelation function is used for drawing out information from the signal of fluctuation. The autocorrelation function used in FCS is shown by the mathematical formula (1).

[Formula 1]

$$C(\tau)=1+\frac{1}{N}\left[\frac{1}{1+\tau/\tau_D}\right]\left[\frac{1}{1+(1/s)^2(\tau/\tau_D)}\right]^{1/2}$$

mathematical formula (1)

Wherein s is s = z/w and indicates a ratio of the radius (w) to half long axis (z) of observing area. $\tau_D$ is called as diffusion time (or correlation time) and indicates average time during which fluorescent molecules pass through the observing area by diffusion. N indicates the average number of molecules existing in the observing area during a constant time.

When the fluctuation is analyzed by the mathematical formula (1), a curve shown in FIG.4(C) is obtained and the diffusion time $\tau_D$ showing the "mobility" of molecules and the "number" N of molecules are obtained thereby. The correlation curve is shifted to right when the size of molecules is enlarged by aggregation of fluorescent molecules with other molecules and the like, and in contrast, is shifted to left when the size is lessened by dissociation and the like.

The diffusion time $\tau_D$ obtained by the mathematical formula (1) is in relation with the diffusion constant D by the mathematical formula (2).

[Formula 2]

$$\tau_D = w^2/4D$$

mathematical formula (2)

Furthermore, the diffusion constant D is in relation shown by the mathematical formula (3) with the radius r of molecule, according to Einstein-Stokes formula in case that the molecule is a sphere.

[Formula 3]

$$D=\frac{\kappa_D T}{6\pi\eta r}$$

mathematical formula (3)

Wherein $\kappa_B$, T and $\eta$ are respectively Boltzmann constant, absolute temperature and the viscosity of solvent.

Accordingly, the diffusion time $\tau_D$ is related in the mathematical formula (4) with the radius of molecule r equivalent to the "size" of molecule by the mathematical formulae (2) and (3).

[Formula 4]

$$\tau_D \propto 1/D \propto r$$

mathematical formula (4)

Thus, the "number" and "sizes" of molecules existing in the observation area can be determined by analyzing the fluctuation of fluorescent intensity, using the mathematical formulae (1) and (4). Detailed illustration with respect to the fluctuation and autocorrelation function is elucidated in an instruction manual ("Blue Backs, The World of Fluctuation" edited by Toshimitsu Musya, published by KODANSYA Ltd. Publishers, 1980; "Physics for Life Science (second volume)"

edited by D. Eizenburg et al, published by BAIFUKAN Co., Ltd., p596-600, 1988; "Spectral Analysis" edited by Mikio Hino, published by Asakura Publishing Co., Ltd., p25-39, (1977)).

In the present invention, a compact FCS manufactured by Hamamatsu Photonics Co. was used as the FCS measurement device. The measurement was carried out by 10-time measurement for 3 seconds at a wavelength of 488 nm. Furthermore, the particle diameter of the water-soluble fluorescent material of the present invention can be measured by carrying out FCS detection and measurement under the same condition described above and using luminescent beads having a publicly known particle diameter as internal standard.

The water-soluble fluorescent material of the present invention is the fluorescent semiconductor nanocrystal having a luminescent peak at visible region and near-infrared region, for example, at 400 to 2000 nm and in particular, 500 to 1200 nm. Furthermore, the water-soluble fluorescent material of the present invention having a structure in which at least one portion on the surface of the semiconductor nanocrystal is coated with a phenol compound has surprising performance that it has higher luminescent efficiency than the luminescent efficiency of the semiconductor nanocrystal used for preparation. Although being not constrained by theory, extremely superior luminescent efficiency of the water-soluble fluorescent material according to the present invention can be provided by coating with a phenol compound having a highly hydrophobic aromatic ring on the surface, which the surface of the semiconductor nanocrystal is highly intercepted from water, therefore exciton electron prepared and charge transfer from the surface of positive hole are suppressed.

Furthermore, additional advantage of the present invention includes a respect that the luminescent wavelength of the water-soluble fluorescent material can be changed by selection of the phenol compound used, without changing the particle diameter of the semiconductor nanocrystal. In general, it is publicly known that the wavelength of luminescent peak of the semiconductor nanocrystal is dependent on the particle diameter of the semiconductor nanocrystal. Accordingly, it has been conventionally required to change the particle diameter of the semiconductor nanocrystal in matching with the wavelength of luminescent peak desired. However, the careful control of the preparation step is required for obtaining the semiconductor nanocrystal having a desired particle diameter. Furthermore, there has been a problem on design because other physical properties such as dispersibility are often changed by changing the particle diameter of the semiconductor nanocrystal.

In the present invention, the luminescent wavelength can be changed to a longer wavelength side at about 60 nm by preparing the water-soluble fluorescent material, using the semiconductor nanocrystal (CdSe(ZnS)) coated with TOPO having a semiconductor nanocrystal core comprising CdSe and a shell layer comprising ZnS, and the acetate derivative of calix[4]arene. The following Table 1 shows the amplitude of shift obtained by comparing the luminescent wavelength of CdSe(ZnS) coated with TOPO, with the luminescent wavelength of the water-soluble fluorescent material prepared by using CdSe(ZnS) and the acetate derivative of calix[4]arene. Furthermore, FIG.5 shows a graph showing the shift of luminescent wavelength of the water-soluble fluorescent material. The phenomenon is caused by that since the semiconductor nanocrystal is coated with an aromatic ring with very high electron density, the electron state of semiconductor is affected to change the size of band gap.

Table 1

| | | | | | |
|---|---|---|---|---|---|
| Luminescent wavelength of CdSe(ZnS) coated with TOPO | 480 nm | 545 nm | 560 nm | 595 nm | 630 nm |
| Amplitude of shift to long wavelength side | 72 nm | 22 nm | 25 nm | 14 nm | 2 nm |

Further advantages of the water-soluble fluorescent material of the present invention include superiority of dispersion stability. It is publicly known that the dispersion stability of a conventional semiconductor nanocrystal is not superior. For example, when the conventional semiconductor nanocrystal dispersed in solution is exposed at room temperature under environmental atmosphere, the semiconductor nanocrystal is precipitated for about 2 weeks and does not emit light. The reason for the phenomenon is probably that a surfactant coating the semiconductor nanocrystal is gradually peeled, the semiconductor nanocrystals are mutually aggregated by Brown motion and form imperfect bond and defects are generated ("Chemical Frontier, Nanomaterial Forefront - Ultimate manufacturing being realized" published by KAGAKU DOZIN Co., Ltd., page 177, columns 11 to 18). On the other hand, the water-soluble fluorescent material of the present invention is superior in dispersion stability in comparison with a conventional semiconductor nanoparticle. The water-soluble fluorescent material of the present invention is superior in dispersion stability, for example, at such level that even if it is dispersed in solution and preserved for about one month or more at room temperature under environmental atmosphere, precipitate is not generated. The water-soluble fluorescent material of the present invention is superior in monodispersibility and is also superior in dispersion stability.

Preparation method of water-soluble fluorescent material

The water-soluble fluorescent material of the present invention is prepared by mixing the above-mentioned semiconductor nanocrystal with the phenol compound. The mixing method is not specifically limited and an arbitrary mixing method can be used.

Ultrasonic processing is preferably carried out in the mixing of the semiconductor nanocrystal with the phenol compound and the water-soluble fluorescent material can be prepared at short time thereby.

The temperature at mixing the semiconductor nanocrystal with the phenol compound is not specifically limited. For example, they can be mixed well even if it is under mild condition such as room temperature. The mixing time can be suitably selected, for example, between 2 seconds and 30 minutes in case of using ultrasonic processing. When the ultrasonic processing is not used, it can be suitably selected, for example, between 10 minutes and 24 hours.

As the solvent used for the mixing, arbitrary organic solvent, the mix solvent of organic solvent and water solvent and the like can be used. The specific example of the organic solvent that can be used includes, for example, aromatic solvents such as toluene and xylene; ketone solvents such as methyl ketone, acetone, methyl isobutyl ketone, and cyclohexanone; ether solvents such as diethyl ether, isopropyl ether, tetrahydrofuran, dioxane, ethyleneglycol dimethyl ether, ethyleneglycol diethyl ether, diethyleneglycol dimethyl ether, diethyleneglycol diethyl ether, propyleneglycol monomethyl ether, anisole and phenetol; ester solvents such as ethyl acetate, butyl acetate, isopropyl acetate and ethylene glycol diatetate; amide solvents such as dimethylformamide, diethylformamide and N-methylpyrrolidone; cellosolve solvents such as methyl cellosolve, ethyl cellosolve and butyl cellosolve; alcohol solvents such as methanol, ethanol and propanol; halogen solvents such as dichloromethane and chloroform, and the like. These organic solvents may be used alone and 2 or more may be used in mixture. Among these, in particular, ketone solvents, ether solvents, ester solvents and the like are preferably used from the viewpoint of workability.

The weight ratio of the semiconductor nanocrystal to the phenol compound used for mixing is dependent on the particle diameter of the semiconductor nanocrystal used, but is generally 1 : 1 to about 1 : 1000 and more preferably 1 : 20 to 1 : 100.

The water-soluble fluorescent material of the present invention and the method for preparing thereof have advantages mentioned below.

1. The water-soluble fluorescent material can be prepared without using a coating agent with toxicity and corrosiveness.
2. Additionally, it can be prepared at short time under mild condition.
3. The water-soluble fluorescent material obtained has excellent luminescent efficiency.
4. The water-soluble fluorescent material obtained is superior in dispersion stability.
5. Luminescent wavelength can be adjusted by changing the kind of a phenol compound used.
6. The water-soluble fluorescent material with monodispersibility (for example, those with a particle diameter of 10 nm or less) can be obtained.
7. The water-soluble fluorescent material of the present invention has superior luminescent efficiency and can be preferably used as a fluorescent probe for biology that is labeled such as protein, antibody and gene.

Examples

The present invention is further specifically illustrated according to Examples but is not limited to these.

Example 1

Preparation of water-soluble fluorescent material using acetate derivative of calix[4]arene

Semiconductor nanocrystal (CdSe(ZnS)) coated with TOPO having a semiconductor nanocrystal core comprising CdSe and a shell layer comprising ZnS was prepared, according to L. Qum and X. Peng. J. Am. Chem. Soc. 124, 2049(2002) and B.O. Dabbousi, J. Rodriguez-Viejo, F.V. Mikulec, J.R. Heine, H. Mattoussi, R. Ober, K.F. Jensen, M.G. Bawendi, J. Phys. Chem. B, 101, 9463(1997). The resultant semiconductor nanocrystal (CdSe(ZnS)) (1 mg) coated with TOPO was dissolved in 4 mL of tetrahydrofuran (THF). To the solution, 20 mg of calix[4]arene acetate derivative shown in the above-mentioned formula (I-1) (wherein n = 1 and $R^3$ = H) was added and ultrasonic treatment was carried out for 30 seconds. Then, 40 mg of potassium t-butoxide was added thereto and stirred. The resultant gel precipitate was separated by centrifugation. To the precipitate obtained, 4 mL of THF was added and centrifuged again to remove excessive calix[4]arene acetate derivative and potassium t-butoxide contained in the precipitate. The precipitate thus obtained was left alone at room temperature for about 10 minutes to remove THF by evaporation. Then, about 5 ml of distilled water was added to the precipitate, ultrasonic treatment was carried out for 5 minutes, and then the mixture was

filtered with a 0.2 micron filter to obtain an aqueous solution of CdSe(ZnS) water-soluble fluorescent material.

The quantum yield of the water-soluble fluorescent material obtained was 0.26 at a luminescent peak of 600 nm by excitation with photo irradiation with wavelength of 480 nm. Furthermore, when 1 mg of the water-soluble fluorescent material obtained was dispersed in 20 mL of water to prepare dispersion liquid and was visually observed after being preserved at room temperature under environmental atmosphere for one month or more, the generation of the precipitate was not confirmed. The result confirmed the superiority of dispersion stability according to the water-soluble fluorescent material.

Fluorescent intensity

The water-soluble fluorescent material (1 mg) obtained in Example 1 was dispersed in 20 mL of water to prepare a sample. Furthermore, 1 mg of the nanocrystal obtained in Comparative Example 1 described later was dispersed in 20 mL of water to prepare a sample, and 1 mg of the CdSe(ZnS) semiconductor nanocrystal coated with TOPO that was used in Example 1 was dispersed in 20 mL of chloroform to prepare a sample. The three samples were diluted with respective solvents so that optical density (absorbance) at 480 nm was 0.05 respectively. Light with a wavelength of 480 nm was irradiated to these samples and the fluorescent intensity of luminescence obtained by this was measured using a fluorescent spectrophotometer RF5300-PC manufactured by Shimadzu Corporation. FIG.6 shows the graph of fluorescent intensity obtained. Furthermore, FIG.7 shows similarly the graph of fluorescent intensity obtained when light with 480 nm was irradiated. In FIGS.6 and 7, (1) shows the fluorescent intensity of the water-soluble fluorescent material of Example 1, (2) shows the fluorescent intensity of nanocrystal of Comparative Example 1 described later and (3) shows the fluorescent intensity of the CdSe(ZnS) semiconductor nanocrystal coated with TOPO that was used in Example 1. The fluorescent intensity (1) of Example 1 and the fluorescent intensity (2) of Comparative Example 1 were measured using the samples that were dispersed in water so that the optical density (absorbance) at 480 nm of the water-soluble fluorescent material was 0.05. On the other hand, the fluorescent intensity (3) of the CdSe(ZnS) semiconductor nanocrystal coated with TOPO was measured using the sample dispersed in chloroform so that the optical density (absorbance) at 480 nm was 0.05.

Dispersion solvents were different in the sample.

The reason why the optical density (absorbance) of respective samples was constant at the measurement of the fluorescent intensity is because molecular weight is not constant by distribution in its size (particle diameter) of the semiconductor nanocrystal. Consequently, absorbance is used as the index of comparison of concentration. When the absorbance of excitation wavelength is same, the number (concentration) of the semiconductor nanocrystal dispersed in solution is also same. Furthermore, the absorbances of the water-soluble fluorescent material of Example 1, the nanocrystal of Comparative Example 1 and the CdSe(ZnS) semiconductor nanocrystal coated with TOPO are estimated to be same since materials coating these have not optical absorption at a longer wavelength than 300 nm.

FIGS. 6 and 7 shows that the fluorescent intensity of the water-soluble fluorescent material in Example 1 is 10-fold or more in comparison with a case using a conventional thiol coating agent in Comparative Example 1.

Measurement of luminescent efficiency

Furthermore, 1 mg of the water-soluble fluorescent material of Example 1 and 1 mg of the nanocrystal of the following Comparative Example 1 were respectively dispersed in 20 ml of water to prepare samples. Furthermore, 1 mg of the CdSe(ZnS) semiconductor nanocrystal coated with TOPO used in Example 1 was dispersed in 20 ml of chloroform to prepare a standard sample. The samples were diluted with respective solvents so that the optical density (absorbance) at 480 nm was 0.05 for each. Then, luminescent spectra obtained by irradiating light with a wavelength of 480 nm on the respective samples were measured and then, the luminescent spectrum of the standard sample in chloroform solvent at the same condition was measured. The intensity (those obtained by integrating luminescent spectrum, namely indicated as an area) of the luminescent spectra obtained by exciting the samples of Example 1 and Comparative Example 1 at a wavelength of 480 nm was determined. Then, the luminescent efficiency was calculated by referring the intensity of the luminescent spectrum of the standard sample under the same condition to as 100%. The result is shown in Table 2.

Table 2

| Example 1 | | Comparative Example | |
|---|---|---|---|
| Luminescent peak | Luminescent efficiency | Luminescent peak | Luminescent efficiency |
| 560nm | 610% | 544nm | 81% |
| 590nm | 516% | 595nm | 60% |

(continued)

| Example 1 | | Comparative Example | |
|---|---|---|---|
| Luminescent peak | Luminescent efficiency | Luminescent peak | Luminescent efficiency |
| 625nm | 180% | 644nm | 71% |

FIGS. 6 and 7 and Table 2 show that luminescent intensity in water of the following Comparative Example 1 is lower than the luminescent intensity in chloroform of the CdSe(ZnS) semiconductor nanocrystal coated with TOPO. Shown in the data, there has been a problem that fluorescent intensity is lowered by dissolving a conventional semiconductor nanocrystal in water. On the other hand, the luminescent intensity in water of the water-soluble fluorescent material in Example 1 exceeds the luminescent intensity in chloroform of the CdSe(ZnS) semiconductor nanocrystal coated with TOPO at any wavelength region. These data can confirm the surprising improvement effect of the luminescent efficiency according to the present invention.

Measurement of particle diameter

The particle diameter of the water-soluble fluorescent material obtained by Example 1 was measured using a compact FCS manufactured by Hamamatsu Photonics Co. The measurement was carried out by measurement 10 times for 3 seconds at a wavelength of 488 nm. The particle diameter of the water-soluble fluorescent material of the present invention was measured by carrying out FCS detection and measurement at the same condition using fluorescent beads (a particle diameter of 14 nm) that are luminescent beads having a publicly known particle diameter and rhodamine 6G (a particle diameter of about 0.5 nm) as internal standard.
FIG.8 shows the measurement result. The result obtained can confirm that the particle diameter of the water-soluble fluorescent material of the present invention is 5 nm or more and 100 nm or less.

Example 2

Preparation of water-soluble fluorescent material using hexyl ether derivative of sulfonated calix[4]arene

Semiconductor nanocrystal (CdSe(ZnS)) coated with TOPO having a semiconductor nanocrystal core comprising CdSe and a shell layer comprising ZnS was prepared, according to L. Qum and X. Peng, J. Am. Chem. Soc. 124, 2049(2002) and B.O. Dabbousi, J. Rodriguez-Viejo, F.V. Mikulec, J.R. Heine, H. Mattoussi, R. Ober, K.F. Jensen, M.G. Bawendi, J. Phys. Chem. B, 101, 9463(1997). The resultant semiconductor nanocrystal (CdSe(ZnS)) (1 mg) coated with TOPO was dissolved in 4 mL of tetrahydrofuran (THF). To the solution, 20 mg of calix[4]arene hexyl ether derivative shown in the above-mentioned formula (II-1) (wherein n = 1 and $R^4$ = $(CH_2)_5CH_3$) was added and ultrasonic treatment was carried out for 5 minutes. The resultant gel precipitate was separated by centrifugation. The precipitate thus obtained was left alone at room temperature for about 10 minutes to remove THF by evaporation. Then, about 5 ml of distilled water was added to the precipitate, ultrasonic treatment was carried out for 5 minutes, and then the mixture was filtered with a 0.2-micron filter to obtain the aqueous solution of CdSe(ZnS) water-soluble fluorescent material.

The water-soluble fluorescent material (1 mg) obtained by Example 2 was dispersed in 20 mL of water to prepare a sample. Furthermore, 1 mg of the nanocrystal obtained in Comparative Example 1 described later was dispersed in 20 mL of water to prepare a sample and 1 mg of the semiconductor nanocrystal (CdSe(ZnS)) coated with TOPO used in Example 1 was dispersed in 20 ml of chloroform to prepare a sample. The three samples were further diluted with respective solvents so that optical density (absorbance) at 480 nm was 0.05 respectively. Light with a wavelength of 480 nm was irradiated to these samples and luminescent spectrum obtained by this was measured using a fluorescent spectrophotometer RF5300-PC manufactured by Shimadzu Corporation. The luminescent intensity of the water-soluble fluorescent material of Example 2 was about 3-fold intensity in comparison with that of Comparative Example 1. Furthermore, the luminescent efficiency of luminescent intensity of the water-soluble fluorescent material was 130% based on the luminescent intensity of the semiconductor nanocrystal (CdSe(ZnS)) coated with TOPO in chloroform.

Furthermore, 1 mg of the water-soluble fluorescent material obtained was dispersed in 20 ml of water to prepare a sample and this was visually observed after being preserved at room temperature under environmental atmosphere for one month or more, the generation of the precipitate was not confirmed. The result confirmed the superiority of dispersion stability according to the water-soluble fluorescent material.

Comparative Example 1

Semiconductor nanocrystal (CdSe(ZnS)) coated with TOPO having a semiconductor nanocrystal core comprising CdSe and a shell layer comprising ZnS was prepared, according to L. Qum and X. Peng, J. Am. Chem. Soc. 124, 2049(2002) and B.O. Dabbousi, J. Rodriguez-Viejo, F.V. Mikulec, J.R. Heine, H. Mattoussi, R. Ober, K.F. Jensen, M.G. Bawendi, J. Phys. Chem. B, 101, 9463(1997). The resultant semiconductor nanocrystal (CdSe(ZnS)) coated with TOPO was coated with mercaptopropionic acid being a thiol compound according to a method described in D. Gerion, F. Pinaud, S.C. Williams, W.J. Parak, D. Zanchet, S. Weiss, and A.P. Alivasatos, J. Phys. Chem. B, 105, 8861 (2001), to obtain nanocrystal dissolved in water.

Example 3

Introduction of GFP antibody into water-soluble fluorescent material of Example 1

Aqueous solution (10 μL) of 1 μM CdSe(ZnS) coated with calix[4]arene acetate derivative was diluted with 70 μL of PBS buffer solution (pH = 7.4). To the solution, 10 μL (5 mg/mL) of EDC (1-ethyl-3-(3-dimethylaminopropyl) carbodiimide) was added, then 10 μL (5 μM) of GFP antibody was added, and the mixture was stirred at room temperature for 1 hour to be labeled. The proceeding situation of labeling reaction was monitored by fluorescent correlation measurement in diffusion time of the semiconductor nanoparticle. FIG.9 is a graph showing the change of dispersion time before and after labeling GFP antibody.
The diffusion time of the water-soluble fluorescent material is nearly 2-fold by labeling the GFP antibody. The result shows that the water-soluble fluorescent material of the present invention can be preferably used for the detection and measurement of antigen protein.
The water-soluble fluorescent material of the present invention can be prepared at short time under mild condition. Furthermore, a coating agent with toxicity and corrosiveness is not required for the preparation. Furthermore, the water-soluble fluorescent material of the present invention has superior luminescent efficiency and is superior in dispersion stability. The water-soluble fluorescent material of the present invention can be used for various uses such as the display field, photonic device field and a fluorescent probe for biology. It can be preferably used as the fluorescent probe for biology labeled such as protein, antibody and gene due to its excellent luminescent efficiency.

## Claims

1. A water-soluble fluorescent material comprising
a semiconductor nanocrystal,
a linear or cyclic phenol compound having hydrophilic group and hydrophobic group that coats at least a portion of the surface of the semiconductor nanocrystal, and
a coordinating organic compound coating at least a portion of the surface of the semiconductor nanocrystal.

2. The water-soluble fluorescent material according to Claim 1, wherein the semiconductor nanocrystal has semiconductor nanocrystal core and a shell layer coating the semiconductor nanocrystal core.

3. The water-soluble fluorescent material according to Claim 1 or 2, wherein the coordinating organic compound is one or more compounds selected from a group consisting of trialkylphosphines, trialkylphosphineoxides and ω-aminoalkanes.

4. The water-soluble fluorescent material according to any one of Claims 1 to 3, wherein the linear or cyclic phenol compound having hydrophilic group and hydrophobic group is one or more calixarene derivatives indicated by the following formula (I):

[Chem. 1]

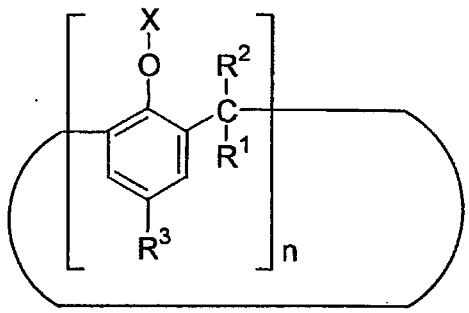

(I)

wherein,
X represents H, $(CH_2)_mCOOH$, $(CH_2)_mOH$, $(CH_2)_mNH_2$, $(CH_2)_mN(CH_3)_3$, $(CH_2)_mPO_4H_2$, $(CH_2)_mSO_3H$, $(CH_2)_mCHO$, $\{(CH_2)_2O\}_m(CH_2)_2OH$ or a salt thereof, wherein m is an integer of 1 to 5, and X may be bonded with other phenolic oxygen atom to form a crown ether with a 3- to 30-membered ring,
each of $R^1$ and $R^2$ independently represents H, $CH_3$, $C_2H_5$ or $C_3H_7$,
$R^3$ represents H, or linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and X may be the same or different,
or indicated by the following formula (II):

[Chem. 2]

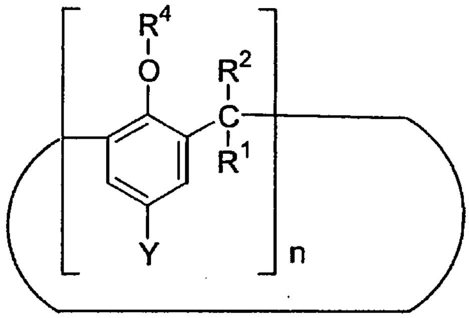

(II)

wherein,
Y represents $(CH_2)_tCOOH$, $(CH_2)_tOH$, $(CH_2)_tNH_2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5,
each of $R^1$ and $R^2$ independently represents H, $CH_3$, $C_2H_5$ or $C_3H_7$, $R^4$ represents linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and Y may be the same or different.

**5.** The water-soluble fluorescent material according to any one of Claims 1 to 3, wherein the linear or cyclic phenol compound having hydrophilic group and hydrophobic group is one or more thiacalixarene derivatives indicated by the following formula (III):

[Chem. 3]

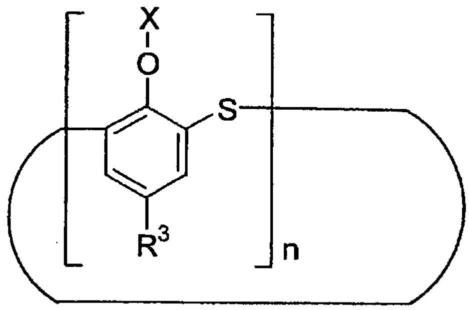

(III)

wherein,
X represents H, $(CH_2)_mCOOH$, $(CH_2)_mOH$, $(CH_2)_mNH_2$, $(CH_2)_mN(CH_3)_3$, $(CH_2)_mPO_4H_2$, $(CH_2)_mSO_3H$, $(CH_2)_mCHO$, $\{(CH_2)_2O\}_m(CH_2)_2OH$ or a salt thereof, wherein m is an integer of 1 to 5 and X may be bonded with other phenolic oxygen atom to form a crown ether with a 3- to 30-membered ring,
$R^3$ represents H, or linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and X may be the same or different,
or indicated by the following formula (IV):

[Chem. 4]

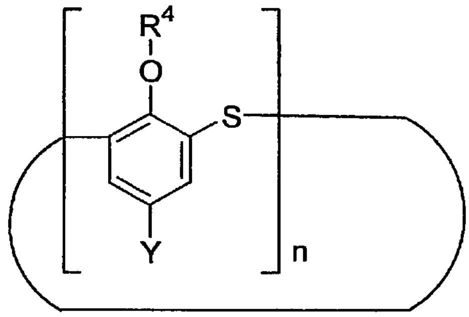

(IV)

wherein,
Y represents $(CH_2)_tCOOH$, $(CH_2)_tOH$, $(CH_2)_tNH_2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5,
$R^4$ represents linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and Y may be the same or different.

6. The water-soluble fluorescent material according to any one of Claims 1 to 3, wherein the linear or cyclic phenol compound having hydrophilic group and hydrophobic group is one or more calixresorcarene derivatives indicated by the following formulae:

[Chem. 5]

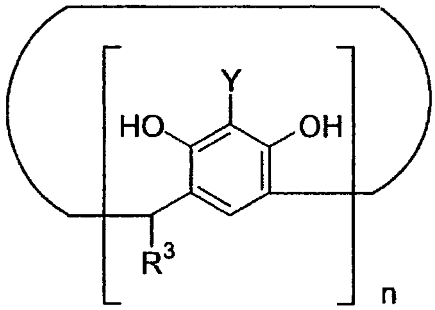

(V)

[Chem. 6]

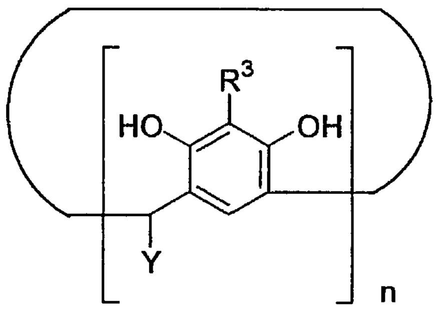

(VI)

[Chem. 7]

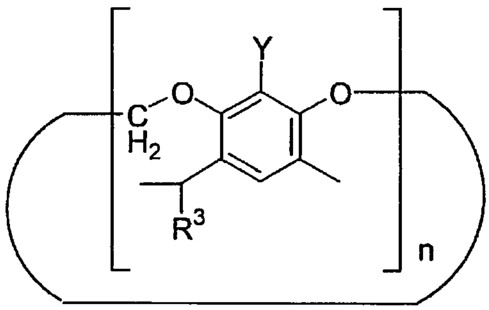

(VII)

[Chem. 8]

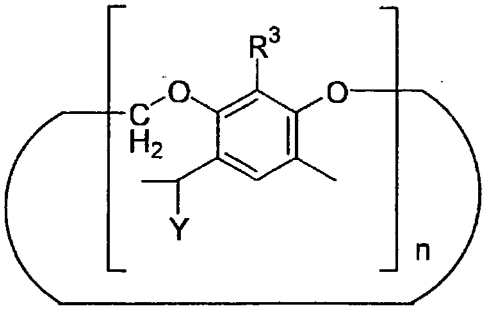

(VIII)

in the formulae (V) to (VIII),

Y represents $(CH_2)_tCOOH$, $(CH_2)_tOH$, $(CH_2)_tNH_2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5,
$R^3$ represents H, or linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and Y may be the same or different.

**7.** The water-soluble fluorescent material according to any one of Claims 1 to 3, wherein the linear or cyclic phenol compound having hydrophilic group and hydrophobic group is one or more linear phenol compound derivatives indicated by the following formula (IX):

[Chem. 9]

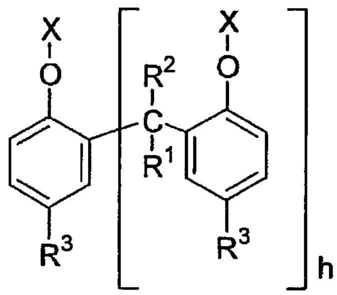

(IX)

wherein,
X represents H, $(CH_2)_mCOOH$, $(CH_2)_mOH$, $(CH_2)_mNH_2$, $(CH_2)_mN(CH_3)_3$, $(CH_2)_mPO_4H_2$, $(CH_2)_mSO_3H$, $(CH_2)_mCHO$, $\{(CH_2)_2O\}_m(CH_2)_2OH$ or a
salt thereof, wherein m is an integer of 1 to 5 and X may be bonded with other phenolic oxygen atom to form a crown ether with a 3- to 30-membered ring,
each of $R^1$ and $R^2$ independently represents H, $CH_3$, $C_2H_5$ or $C_3H_7$,
$R^3$ represents H, or linear or branched $C_1$ to $C_{12}$-alkyl, $C_3$ to $C_{12}$-cycloalkyl, $C_6$ to $C_{12}$-aryl or $C_7$ to $C_{12}$-aralkyl,
h is an integer of 0 to 99 and if h is 1 or more, X may be the same or different,
or indicated by the following formula (X):

[Chem. 10]

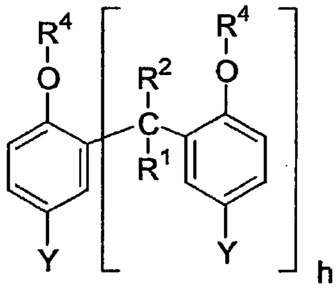

(X)

wherein,
Y represents $(CH_2)_tCOOH$, $(CH_2)_tOH$, $(CH_2)_tNH_2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5,
each of $R^1$ and $R^2$ independently represents H, $CH_3$, $C_2H_5$ or $C_3H_7$, $R^4$ represents linear or branched $C_1$ to $C_{12}$-alkyl, $C_3$ to $C_{12}$-cycloalkyl, $C_6$ to $C_{12}$-aryl or $C_7$ to $C_{12}$-aralkyl,
h is an integer of 0 to 99 and if h is 1 or more, Y may be the same or different.

**8.** The water-soluble fluorescent material according to any one of Claims 2 to 7, wherein the semiconductor nanocrystal core comprises Group II-VI compound semiconductor, Group III- V compound semiconductor or Group IV semiconductor.

**9.** The water-soluble fluorescent material according to any one of Claims 2 to 8, wherein the shell layer comprises one or more semiconductors selected from a group consisting of ZnO, ZnS, ZnSe, ZnTe, CdO, CdS, CdSe, CdTe, MgS, MgSe, GaAs, GaN, GaP, GaAs, GaSb, HgO, HgS, HgSe, HgTe, InAs, InN, InP, InSb, AlAs, AlN, AlP and AlSb, or an alloy or mixed crystal thereof.

**10.** The water-soluble fluorescent material according to any one of Claims 2 to 7, wherein the semiconductor nanocrystal core comprises CdSe or CdTe, or a mixture, an alloy or mixed crystal thereof, and wherein the shell layer comprises ZnS.

**11.** A method for preparing a water-soluble fluorescent material comprising a step of mixing a semiconductor nanocrystal whose surface is coated with a coordinating organic compound, with a linear or cyclic phenol compound having hydrophilic group and hydrophobic group under the presence of solvent.

**12.** The method for preparing the water-soluble fluorescent material according to Claim 11, wherein the semiconductor nanocrystal has a semiconductor nanocrystal core and a shell layer coating the semiconductor nanocrystal core.

**13.** The method for preparing the water-soluble fluorescent material according to Claims 11 or 12, wherein the linear or cyclic phenol compound having hydrophilic group and hydrophobic group used in the mixing step is one or more calixarene derivatives indicated by the following formula (I) :

[Chem. 11]

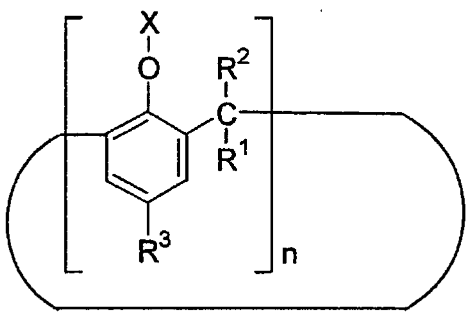

(I)

wherein,
X represents H, $(CH_2)_mCOOH$, $(CH_2)_mOH$, $(CH_2)_mNH_2$, $(CH_2)_mN(CH_3)_3$, $(CH_2)_mPO_4H_2$, $(CH_2)_mSO_3H$, $(CH_2)_mCHO$, $\{(CH_2)_2O\}_m(CH_2)_2OH$ or a
salt thereof, wherein m is an integer of 1 to 5 and X may be bonded with other phenolic oxygen atom to form a crown ether with a 3- to 30-membered ring,
each of $R^1$ and $R^2$ independently represents H, $CH_3$, $C_2H_5$ or $C_3H_7$,
$R^3$ represents H, or linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and X may be the same or different,
or indicated by the following formula (II):

[Chem. 12]

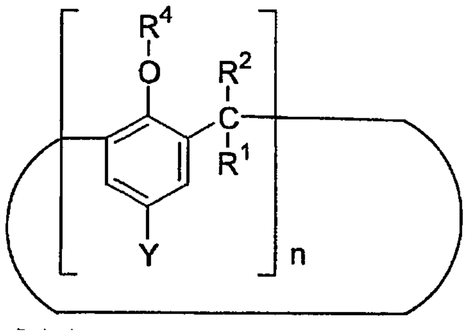

(II)

wherein,
Y represents $(CH_2)_tCOOH$, $(CH_2)_tOH$, $(CH_2)_tNH_2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5,
each of $R^1$ and $R^2$ independently represents H, $CH_3$, $C_2H_5$ or $C_3H_7$, $R^4$ represents linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and Y may be the same or different, or
one or more thiacalixarene derivatives indicated by the following formula (III):

[Chem. 13]

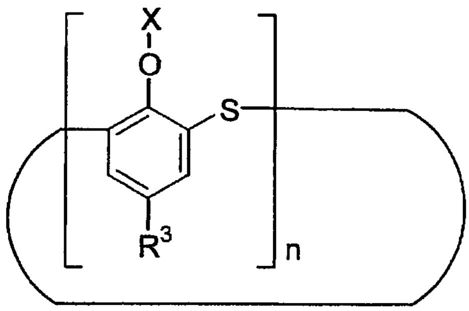

(III)

wherein,
X represents H, $(CH_2)$,COOH, $(CH_2)_mOH$, $(CH_2)_mNH_2$, $(CH_2)_mN(CH_3)_3$, $(CH_2)_mPO_4H_2$, $(CH_2)_mSO_3H$, $(CH_2)_mCHO$, $\{(CH_2)_2O\}_m(CH_2)_2OH$ or a
salt thereof, wherein m is an integer of 1 to 5 and X may be bonded with other phenolic oxygen atom to form a crown ether with a 3- to 30-membered ring,
$R^3$ represents H, or linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and X may be the same or different,
or indicated by the following formula (IV):

[Chem. 14]

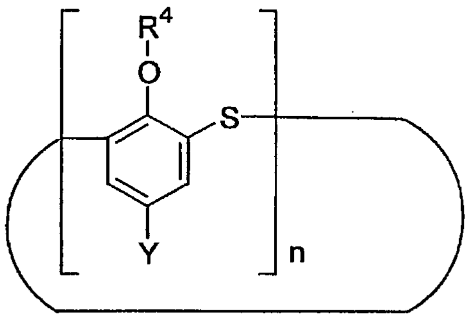

(IV)

wherein,
Y represents $(CH_2)_tCOOH$, $(CH_2)_tOH$, $(CH_2)_tNH2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5,
$R^4$ represents linear or branched $C_1$ to $C_{12}$-alkyl, $C_3$ to $C_{12}$-cycloalkyl, $C_6$ to $C_{12}$-aryl or $C_7$ to $C_{12}$-aralkyl,
n is an integer of 4 to 20 and Y may be the same or different, or
one or more calixresorcarene derivatives indicated by the following formulae:

[Chem. 15]

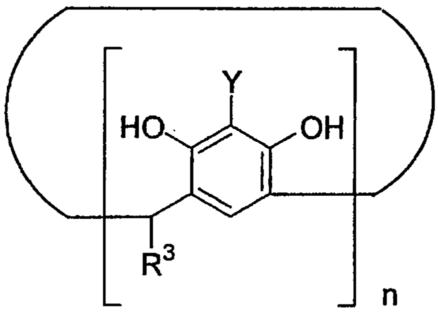

(V)

[Chem. 16]

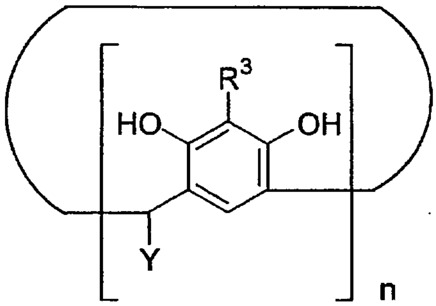

(VI)

[Chem. 17]

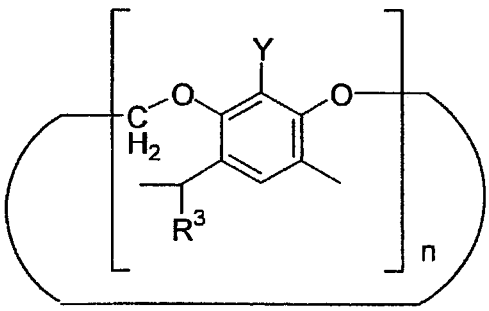

(VII)

[Chem. 18]

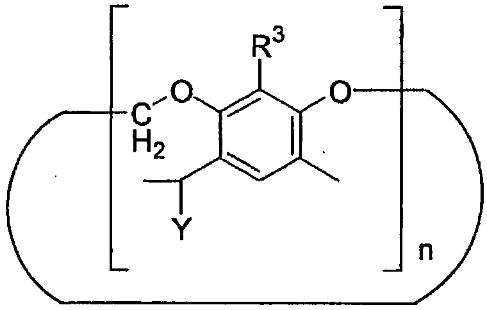

(VIII)

in the formulae (V) to (VIII),

Y represents $(CH_2)_tCOOH$, $(CH_2)_tOH$, $(CH_2)_tNH_2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5,

$R^3$ represents H, or linear or branched $C_1$ to $C_{12}$-alkyl, $C_3$ to $C_{12}$-cycloalkyl, $C_6$ to $C_{12}$-aryl or $C_7$ to $C_{12}$-aralkyl,

n is an integer of 4 to 20 and Y may be the same or different,

or

one or more linear phenol compound derivatives indicated by the following formula (IX):

[Chem. 19]

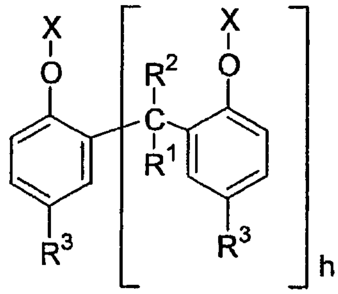

(IX)

wherein,

X represents H, $(CH_2)_mCOOH$, $(CH_2)_mOH$, $(CH_2)_mNH_2$, $(CH_2)_mN(CH_3)_3$, $(CH_2)_mPO_4H_2$, $(CH_2)_mSO_3H$, $(CH_2)_mCHO$, $\{(CH_2)_2O\}_m(CH_2)_2OH$ or a salt thereof, wherein m is an integer of 1 to 5 and X may be bonded with other phenolic oxygen atom to form a crown ether with a 3- to 30-membered ring,

each of $R^1$ and $R^2$ independently represents H, $CH_3$, $C_2H_5$ or $C_3H_7$,

$R^3$ represents H, or linear or branched $C_1$ to $C_{12}$-alkyl, $C_3$ to $C_{12}$-cycloalkyl, $C_6$ to $C_{12}$-aryl or $C_7$ to $C_{12}$-aralkyl,

h is an integer of 0 to 99 and if h is 1 or more, X may be the same or different,

or indicated by the following formula (X):

[Chem. 20]

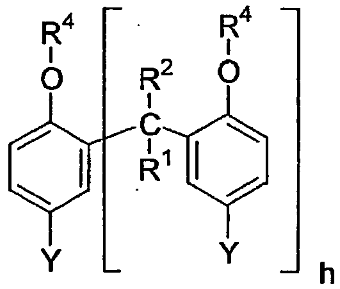

(X)

wherein,
Y represents $(CH_2)_tCOOH$, $(CH_2)_tOH$, $(CH_2)_tNH_2$, $(CH_2)_tN(CH_3)_3$, $(CH_2)_tPO_4H_2$, $(CH_2)_tSO_3H$, $(CH_2)_tCHO$, $\{(CH_2)_2O\}_t(CH_2)_2OH$ or a salt thereof, wherein t is an integer of 0 to 5,
each of $R^1$ and $R^2$ independently represents H, $CH_3$, $C_2H_5$ or $C_3H_7$, $R^4$ represents linear or branched $C_1$ to $C_{12}$-alkyl, $C_3$ to $C_{12}$-cycloalkyl, $C_6$ to $C_{12}$-aryl or $C_7$ to $C_{12}$-aralkyl,
h is an integer of 0 to 99 and if h is 1 or more, Y may be the same or different.

**14.** The method for preparing the water-soluble fluorescent material according to any one of Claims 11 to 13, wherein the semiconductor nanocrystal core comprises Group II-VI compound semiconductor, Group III- V compound semiconductor or Group IV semiconductor.

**15.** The method for preparing the water-soluble fluorescent material according to any one of Claims 11 to 14, wherein the shell layer comprises one or more semiconductors selected from a group consisting of ZnO, ZnS, ZnSe, ZnTe, CdO, CdS, CdSe, CdTe, MgS, MgSe, GaAs, GaN, GaP, GaAs, GaSb, HgO, HgS, HgSe, HgTe, InAs, InN, InP, InSb, AlAs, AlN, AlP and AlSb, or an alloy or mixed crystal thereof.

**16.** The method for preparing the water-soluble fluorescent material according to any one of Claims 11 to 13, wherein the semiconductor nanocrystal core comprises CdSe or CdTe, or a mixture, an alloy or mixed crystal thereof, and wherein the shell layer comprises ZnS.

**17.** The method for preparing the water-soluble fluorescent material according to any one of Claims 11 to 16, wherein ultrasonic processing is used in the mixing step.

**18.** The method for preparing the water-soluble fluorescent material according to any one of Claims 11 to 17, for obtaining the water-soluble fluorescent material having a luminescent region at visible and near-infrared range.

**19.** The method for preparing the water-soluble fluorescent material according to any one of Claims 11 to 17, for obtaining the water-soluble fluorescent material having a luminescent region at visible range.

**20.** The water-soluble fluorescent material obtainable form the method according to any one of Claims 11 to 19.

**21.** Use of a linear or cyclic phenol compound having hydrophilic group and hydrophobic group in the preparation of the water-soluble fluorescent material according to any one of Claims 1 to 10.

**22.** A method for preparing a water-soluble fluorescent material having a changed luminescent wavelength, comprising a step of mixing a semiconductor nanocrystal whose surface is coated with a coordinating organic compound, with a linear or cyclic phenol compound having hydrophilic group and hydrophobic group in solvent.

Fig. 1

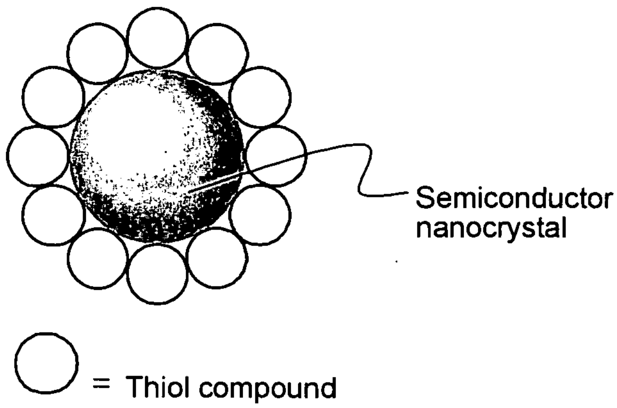
Semiconductor
nanocrystal
= Thiol compound

Fig. 2

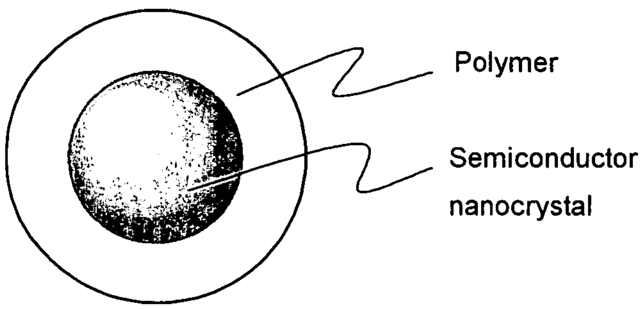
Polymer
Semiconductor
nanocrystal

Fig. 3

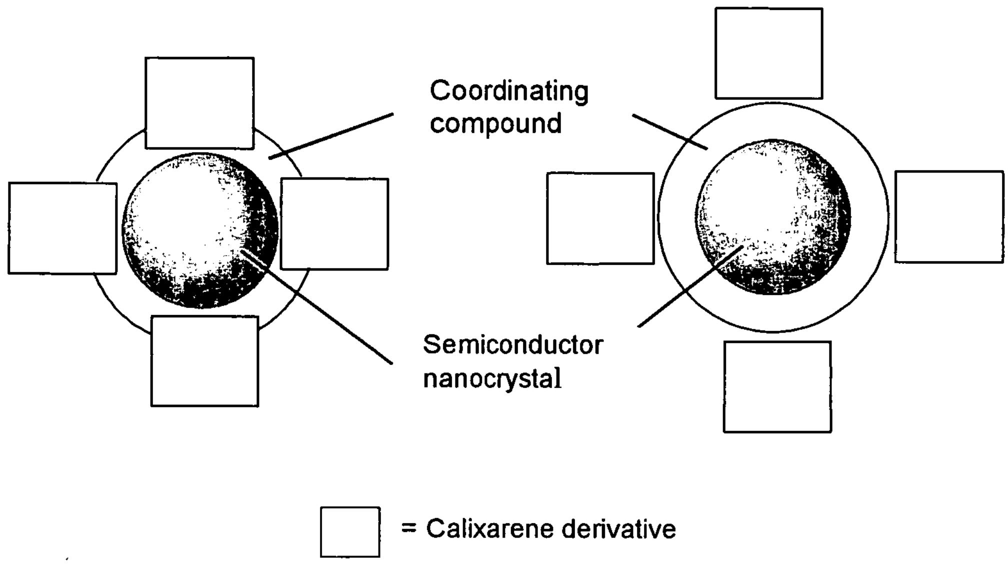
Coordinating
compound
Semiconductor
nanocrystal
= Calixarene derivative

Fig. 4

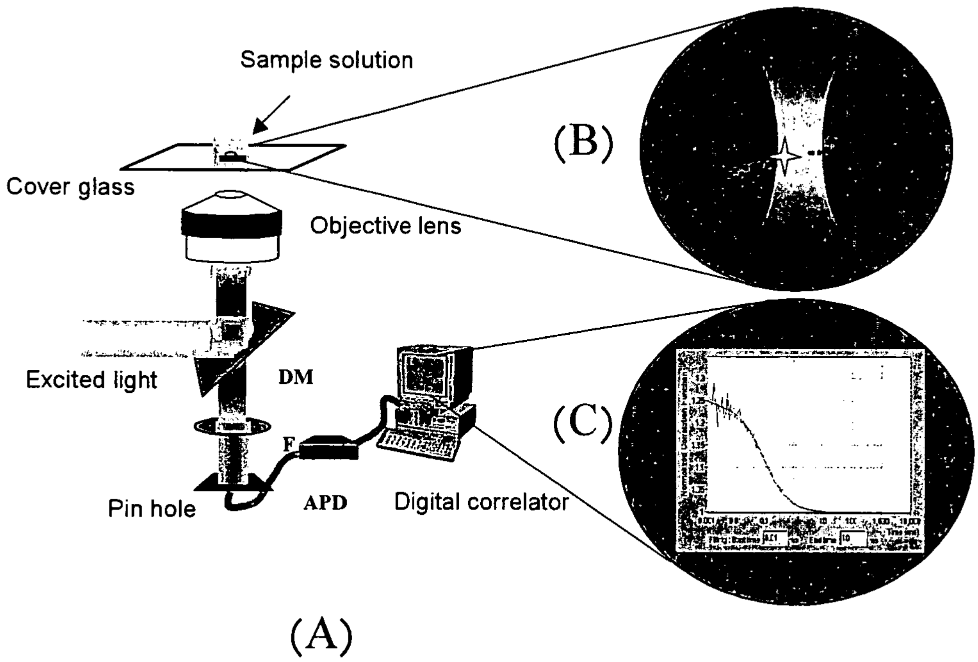
Sample solution
Cover glass
Objective lens
(B)
Excited light
DM
(C)
F
Pin hole
APD
Digital correlator
(A)

Fig. 5

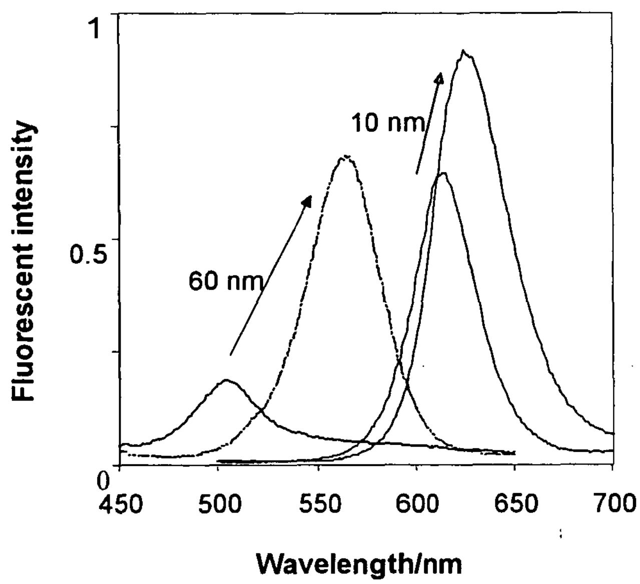

1
0.5
0
Fluorescent intensity
10 nm
60 nm
450
500
550
600
650
700
Wavelength/nm


Fig. 6

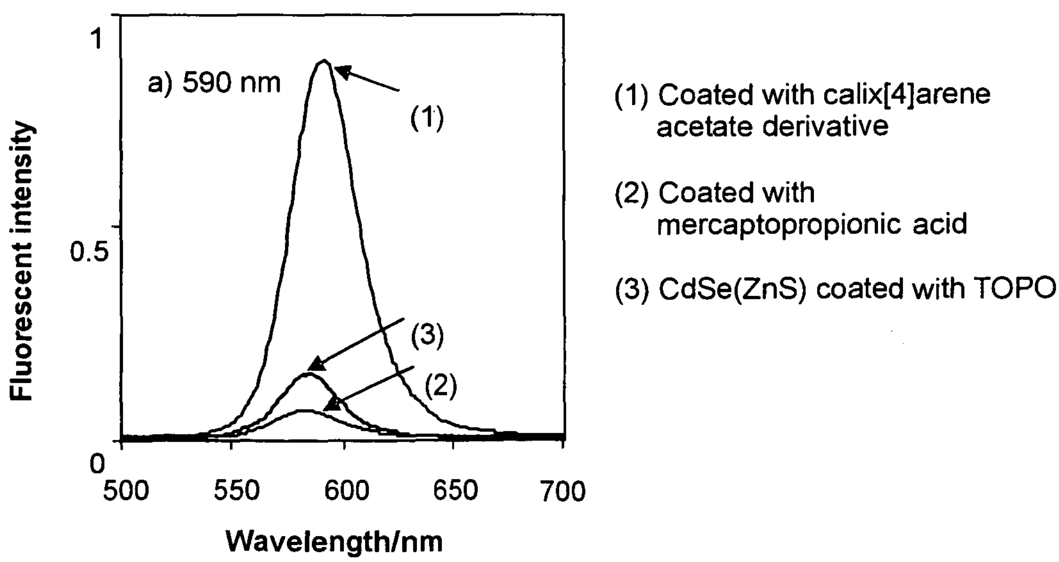

1
0.5
0
Fluorescent intensity
a) 590 nm
(1)
(3)
(2)
500
550
600
650
700
Wavelength/nm
(1) Coated with calix[4]arene acetate derivative
(2) Coated with mercaptopropionic acid
(3) CdSe(ZnS) coated with TOPO

Fig. 7

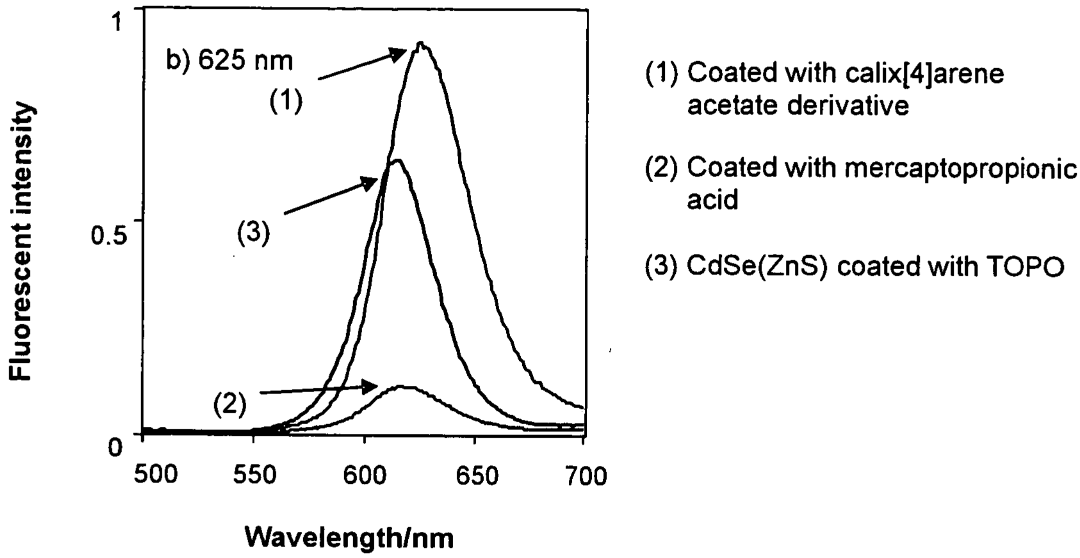
1
0.5
0
Fluorescent intensity
b) 625 nm
(1)
(3)
(2)
500
550
600
650
700
Wavelength/nm
(1) Coated with calix[4]arene acetate derivative
(2) Coated with mercaptopropionic acid
(3) CdSe(ZnS) coated with TOPO

Fig. 8

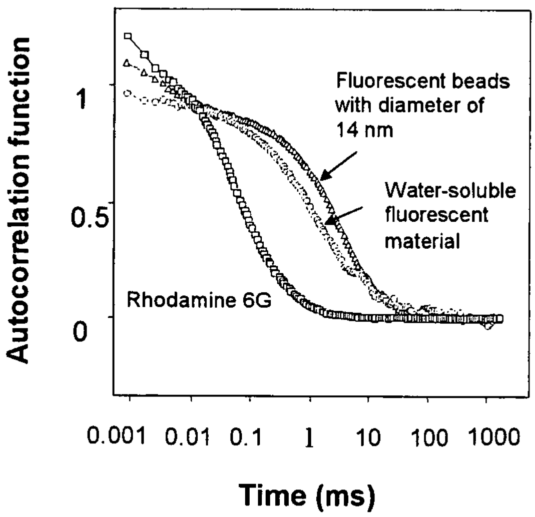
Autocorrelation function
1
0.5
0
Fluorescent beads with diameter of 14 nm
Water-soluble fluorescent material
Rhodamine 6G
0.001
0.01
0.1
1
10
100
1000
Time (ms)

Fig. 9

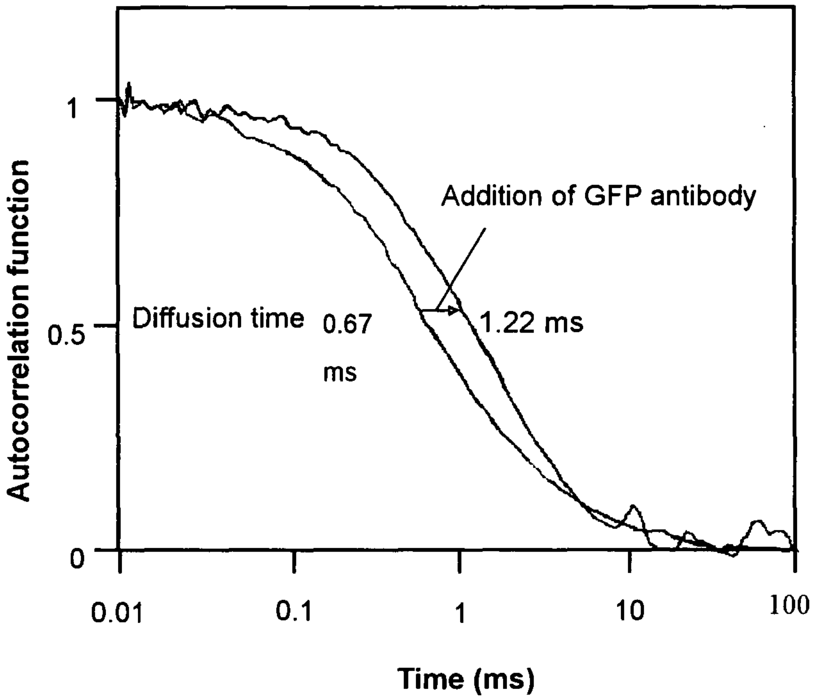
1
0.5
0
Autocorrelation function
Addition of GFP antibody
Diffusion time 0.67 ms
1.22 ms
0.01
0.1
1
10
100
Time (ms)

**INTERNATIONAL SEARCH REPORT**

International application No.
PCT/JP2005/017493

A. CLASSIFICATION OF SUBJECT MATTER
*C09K11/08*(2006.01), *C09K11/56*(2006.01), *C09K11/62*(2006.01), *C09K11/64*(2006.01), *C09K11/70*(2006.01), *C09K11/74*(2006.01), *C09K11/88*(2006.01)

According to International Patent Classification (IPC) or to both national classification and IPC

B. FIELDS SEARCHED

Minimum documentation searched (classification system followed by classification symbols)
C09K11/00-11/89

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 00/17655 A1 (MASSACHUSETTS INSTITUTE OF TECHNOLOGY), 30 March, 2000 (30.03.00), Full text & JP 2002-525394 A & JP 2003-523718 A & JP 2003-524147 A & US 6251303 B1 & US 6326144 B1 & US 6602671 B1 & US 6306610 B1 & EP 990903 A1 | 1-22 |
| A | JP 2002-129156 A (Mitsubishi Chemical Corp.), 09 May, 2002 (09.05.02), Full text (Family: none) | 1-22 |

☐ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 26 December, 2005 (26.12.05) | 17 January, 2006 (17.01.06) |
| Name and mailing address of the ISA/ Japanese Patent Office | Authorized officer |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0017655 A


### Non-patent literature cited in the description


- **W.C.W. CHAN ; S. NIE.** *Science,* 1998, vol. 281, 2016
- **D. GERION ; F. PINAUD ; S.C. WILLIAMS ; W.J. PARAK ; D. ZANCHET ; S. WEISS ; A.P. ALIVASATOS.** *J. Phys. Chem. B.,* 2001, vol. 105, 8861
- **B. DUBERTRET ; P. SKOURIDES ; D.J. NORRIS ; V. NOIREAUX ; A.H. BRIVANLOU ; A. LIBCHABER.** *Science,* 2002, vol. 298, 1759
- **S. KIM ; Y.T. LIM ; E.G. SOLTESZ ; A.M. DE GRAND ; J. LEE ; A. NAKAYAMA ; J.A. PARFER ; T. MIHALJEVIC ; R.G. LAURENCE ; D.M. DOR.** *Nature Biotech.,* 2004, vol. 22, 93
- **B.S. ZOU et al.** *Int. J. Quant. Chem.,* 1999, vol. 72, 439
- **L. QUM ; X. PENG.** *J. Am. Chem. Soc.,* 2002, vol. 124, 2049
- *J. Am. Chem. Soc.,* 1989, vol. 111, 8192
- *Macromol. Chem. Rapid Commun.,* 1982, vol. 3, 705
- *J. Org. Chem.,* 1977, vol. 42, 382
- *J. Org. Chem.,* 1978, vol. 43, 4280
- **A. ARUDUINI ; A. POCHINI ; S. REVERBERI ; R. UNGARO.** *J. Chem. Soc. Commun.,* 1984, 981-982
- **S.SHINKAI ; S. MORI ; H. KOREISHI ; T. TSUBAKI ; O. MANABE.** *J. Am. Chem. Soc.,* 1986, vol. 108, 2409-2416
- *Protein, Nucleic Acid And Enzyme,* 1999, vol. 9, 1431-1438
- Blue Backs, The World of Fluctuation. KODANSYA Ltd. Publishers, 1980
- Physics for Life Science. BAIFUKAN Co., Ltd, 1988, 596-600
- Spectral Analysis. Asakura Publishing Co., Ltd, 1977, 25-39
- **B.O. DABBOUSI ; J. RODRIGUEZ-VIEJO ; F.V. MIKULEC ; J.R. HEINE ; H. MATTOUSSI ; R. OBER ; K.F. JENSEN ; M.G. BAWENDI.** *J. Phys. Chem. B,* 1997, vol. 101, 9463
- **D. GERION ; F. PINAUD ; S.C. WILLIAMS ; W.J. PARAK ; D. ZANCHET ; S. WEISS ; A.P. ALIVASATOS.** *J. Phys. Chem. B,* 2001, vol. 105, 8861